# EUROPEAN PATENT APPLICATION

(11) **EP 4 389 748 A2**
(43) Date of publication of application: **26.06.2024**
(21) Application number: 21828682.1
(22) Date of filing: 08.10.2021
(51) Int. Cl.: C07D 471/08, A61K 31/55, A61K 9/20, A61P 1/00, A61P 25/34, A01N 51/00

(54) **NITROSAMINE IMPURITY, VARENICLINE PHARMACEUTICAL COMPOSITION CAPABLE OF REDUCING GENERATION OF NITROSAMINE IMPURITIES AND PREPARATION AND USE THEREOF**

(30) Priority: 20.08.2021 CN 202110961274; 27.08.2021 CN 202110997332
(71) Applicant: Viwit Pharmaceutical Co., Ltd., Tengzhou, Shandong 277500 (CN); Shandong Weizhi Baike Pharmaceutical Co., Ltd., Zaozhuang, Shandong 277000 (CN); Shandong Weizhi Zhongke Pharmaceutical Co., Ltd., Zaozhaung, Shandong 277500 (CN)
(72) Inventor: WEI, Yanjun, Zaozhuang, Shandong 277500 (CN); BASAVARAJ, Shidagonnavar, Zaozhuang, Shandong 277000 (CN); LI, Feng, Zaozhuang, Shandong 277000 (CN); SUN, Zhongya, Zaozhuang, Shandong 277000 (CN); PEI, Hui, Zaozhuang, Shandong 277500 (CN); DU, Wenfeng, Zaozhuang, Shandong 277500 (CN); WANG, Jian, Zaozhuang, Shandong 277500 (CN); KONG, Meng, Zaozhuang, Shandong 277500 (CN); LIU, Xiwang, Zaozhaung, Shandong 277500 (CN); XING, Yanping, Zaozhaung, Shandong 277500 (CN); XU, Qingjing, Zaozhuang, Shandong 277000 (CN)
(74) Representative: Plasseraud IP
(86) International application number: PCT/CN2021/122654
(87) International publication number: WO 2021/259396

(57) **Abstract**

The present disclosure discloses nitrosamine impurities, a varenicline pharmaceutical composition capable of reducing the generation of nitrosamine impurities, and the preparation and use thereof. By means of adding a pharmaceutically acceptable acid to a secondary amine compound or a composition thereof, the pharmaceutical composition of the present disclosure can effectively inhibit and reduce the generation of nitrosamine impurities, improve the stability of the secondary amine compound or the composition thereof, and control the content of genotoxic nitrosamine impurities to be at a relatively low level, so as to comply with safety requirements.

## Description

The present application claims the priority of Chinese patent application 2021109612747 filed on August 20, 2021 and Chinese patent application 2021109973321 filed on August 27, 2021. The Chinese patent application CN2021109612747 and CN2021109973321 are incorporated herein by reference in their entirety.

### FIELD OF THE DISCLOSURE

The present disclosure belongs to pharmaceutical field, and specifically relates to a nitrosamine impurity, a varenicline pharmaceutical composition capable of reducing the generation of nitrosamine impurities, and the preparation and use thereof.

### BACKGROUND OF THE DISCLOSURE

Varenicline is an aryl fused azapolycyclic compound, which is useful in regulating cholinergic action and is sold in the form of the tartrate salt under the trade name of Chantix^{®} or Champix^{®}. Varenicline can bind to the neuronal nicotinic acetylcholine specific receptor sites and is useful in the treatment of following diseases: inflammatory bowel disease (including but not limited to ulcerative colitis, pyoderma gangrenosum and Crohn's disease), irritable bowel syndrome, spastic dystonia, chronic pain, acute pain, celiac sprue, pouchitis, vasoconstriction, anxiety, panic disorder, depression, bipolar disorder, autism, sleep disorders, jet lag, amyotrophic lateral sclerosis (ALS), cognitive dysfunction, drug/toxin-induced cognitive impairment (e.g., from alcohol, barbiturates, vitamin deficiencies, recreational drugs, lead, arsenic, mercury), diseaseinduced cognitive impairment (e.g., arising from Alzheimers disease (senile dementia), vascular dementia, Parkinson's disease, multiple sclerosis, AIDS, encephalitis, trauma, renal and hepatic encephalopathy, hypothyroidism, Pick's disease, Korsakoff's syndrome and frontal and subcortical dementia), hypertension, bulimia, anorexia, obesity, cardiac arrhythmias, gastric acid hypersecretion, ulcers, pheochromocytoma, progressive supramuscular palsy, chemical dependencies and addictions (e.g., dependencies on, or addictions to nicotine (and/or tobacco products), alcohol, benzodiazepines, barbiturates, opioids or cocaine), headache, migraine, stroke, traumatic brain injury (TBI), obsessive-compulsive disorder (OCD), psychosis, Huntington's chorea, tardive dyskinesia, hyperkinesia, dyslexia, schizophrenia, multi-infarct dementia, age-related cognitive decline, epilepsy, including petit mal absence epilepsy, attention deficit hyperactivity disorder (ADHD), Tourette's Syndrome, particularly, nicotine dependency, addiction and withdrawal; including use in smoking cessation therapy (see, CN1509174A).

Nitrosamine compounds, containing the nitroso group (N (R1) (R2)-N=O), are the genotoxic substances to some animals, and some of them have been classified as the probable or potential human carcinogen by the International Agency for Research on Cancer (IARC). These nitrosamine compounds, called as "cohort of concern" compounds in the guidance document M7 (R1) of the International Conference on Harmonization of Technical Requirements for Pharmaceuticals for Human Use (ICH), are used to assess and control DNA reactive (mutagenic) impurities in pharmaceuticals to limit potential carcinogenic risk (March 2018). ICH guidelines recommend that any known mutagen/cancerogen, including the nitrosamine compounds, should be controlled at intake levels where the risk of cancer in humans is negligible.

At present, Food and Drug Administration (FDA) has identified seven nitrosamine impurities that could theoretically be present in drugs due to the use of preparation processes and materials that could lead to the formation of nitrosamines: N, N-nitrosodimethylamine (NDMA), N-nitroso-diethyl amine (NDEA), N-nitroso-N-methyl-4-aminobutyric acid (NMBA), N-nitroso-isopropyl ethylamine (NIPEA), N-nitroso-diisopropylamine (NIDPA), N-nitroso-dibutylamine (NDBA), and N-nitroso-methyl aniline (NMPA). Five impurities (NDMA, NDEA, NMBA, NIPEA and NMPA) have been detected from active pharmaceutical ingredients (API) or drugs.

For example, some of preliminary results from FDA indicated that the contents of NDMA in some ranitidine products have exceeded the acceptable level. In recent years, NDMA impurities have been detected from some metformin products by FDA, and the contents of the NDMA detected in some batches are higher than the acceptable intake limits recommended by FDA.

At present, some studies have tried to control and reduce the generation of nitrosamine impurities via technical means, for example: CN 109748905A discloses that the sodium nitrite is replaced with hydrogen dioxide to reduce the contents of NDMA and NDEA in the sartan APIs; CN 111686087A discloses that coating is carried out by the process of direct pressure of mixed powder and coating premix of stomach-soluble film to avoid excessive heat generation in the production process and reduce the risk of generating NDMA; CN 113081990A discloses that the genotoxic impurity N-nitrosodimethylamine (NDMA) is controlled by controlling the content of the impurity dimethylamine in the API metformin hydrochloride and the content of the impurity nitrite in the excipient hydroxypropyl methylcellulose (HPMC).

Specifically in the present disclosure, there may also be a problem that the content of nitrosamine impurities may exceed the standard for varenicline or pharmaceutically acceptable salt, crystal form, solvate, composition, formulation thereof and other pharmaceutical compounds.

Thus, it is necessary to detect and control the content of nitrosamine impurities in varenicline or pharmaceutically acceptable salt, crystal form, solvate, composition, and formulation thereof, and in other pharmaceutical compounds.

In the view of above, the present disclosure is hereby proposed.

### SUMMARY OF THE DISCLOSURE

In order to solve the technical problems and/or deficiencies in the prior arts, one of the purposes of the present disclosure lies in providing a pharmaceutical composition of a secondary amine compound (varenicline or pharmaceutically acceptable salt thereof) capable of reducing the generation of nitrosamine impurities. By means of adding a pharmaceutically acceptable acid to a secondary amine compound or the composition thereof, the generation of nitrosamine impurities in the above pharmaceutical composition can be effectively inhibited and reduced, the stability of the secondary amine compound or the composition thereof can be improved, and the content of genotoxic nitrosamine impurities can be controlled at a low level, thereby complying with the safety requirements.

The present disclosure provides a pharmaceutical composition, comprising: a secondary amine compound and a pharmaceutically acceptable acid; wherein the secondary amine compound is varenicline (including tautomeric isomers, stereoisomers, and the like) or a pharmaceutically acceptable salt thereof.

Further, in any of the above technical solutions (pharmaceutical composition), the secondary amine compound is the pharmaceutically acceptable salt of varenicline; preferably, the secondary amine compound is varenicline hydrochloride or varenicline tartrate.

Further, in any of the above technical solutions (pharmaceutical composition), the secondary amine compound and the pharmaceutically acceptable acid are at a molar ratio of 1: (0.01-50) (for example, the molar ratio of 1:0.05, 1:0.1, 1:0.5, 1:1, 1:1.5, 1:2, 1:2.5, 1:3, 1:4, 1:5, 1:6, 1:7, 1:8, 1:9, 1:10, 1:15, 1:20, 1:25, 1:30 or 1:40, and the like); preferably, the secondary amine compound and the pharmaceutically acceptable acid are at a molar ratio of 1: (1-20); more preferably, the secondary amine compound and the pharmaceutically acceptable acid are at a molar ratio of 1: (2-10).

Further, in any of the above technical solutions (pharmaceutical composition), preferably, the active ingredient of the pharmaceutical composition is varenicline.

Further, in any of the above technical solutions (pharmaceutical composition), the pharmaceutical composition further comprises pharmaceutically acceptable excipients; preferably, the pharmaceutically acceptable excipients comprise one or more of the following excipients (1) - (5):
(1) Diluent: the diluent is selected from the group consisting of one or more of microcrystalline cellulose, silicified microcrystalline cellulose, dibasic calcium phosphate (including anhydrous substance and hydrate), mannitol, copovidone, hydroxypropyl cellulose, hydroxypropyl methyl cellulose, methyl cellulose, ethyl cellulose, starch, maltodextrin, agar, and guargum; preferably, the diluent comprises microcrystalline cellulose and/or anhydrous dibasic calcium phosphate; more preferably, the diluent is microcrystalline cellulose and anhydrous dibasic calcium phosphate; still further preferably, the active ingredient and the diluent are at a weight ratio of 1: (100-300); preferably of 1: (150-250); more preferably of 1: (184-195);
(2) Disintegrant: the disintegrant is selected from the group consisting of one or more of croscarmellose sodium, sodium starch glycolate, crospovidone, partially prepasted starch, partially pregelatinized starch, pregelatinized starch, pregelatinized hydroxypropyl starch, sodium carboxymethyl cellulose, and calcium carboxymethyl cellulose; preferably, the disintegrant is partially pregelatinized starch; more preferably, the active ingredient and the disintegrant are at a weight ratio of 1: (1-10); preferably, of 1: (2-6); more preferably, of 1: (3.5-4.5); for example, of 1:4;
(3) Glidant: the glidant is selected from the group consisting of one or more of colloidal silicon dioxide, fumed silicon dioxide, colloidal silica, corn starch, talc, calcium silicate, magnesium silicate, calcium phosphate tribasic, and silicone hydrogel; preferably, the glidant is colloidal silicon dioxide; more preferably, the active ingredient and the glidant are at a weight ratio of 1: (0.1-8); preferably, of 1: (0.2-5); more preferably, of 1: (0.5-2.5); most preferably of 1: (0.8-2); for example, of 1:0.8, 1:1, or 1:2;
(4) Lubricant: the lubricant is selected from the group consisting of one or more of stearic acid, stearate, talc, mineral oil, malt, glyceryl monostearate, glyceryl benzoate, glyceryl stearate palmitin, hydrogenated vegetable oil, polyethylene glycol, sodium benzoate, sodium acetate, sodium chloride, leucine, lauryl magnesium sulfate, and lauryl sodium sulfate; preferably, the lubricant is stearic acid or stearate, and the stearate is magnesium stearate or sodium stearate; more preferably, the active ingredient and the lubricant are at a weight ratio of 1: (0.1-8); preferably, of 1: (0.2-5); more preferably, of 1: (0.5-2.5); most preferably of 1: (1-2); for example, 1:1, 1:1.4, or 1:2;
(5) Coating agent: preferably, the coating agent is Opadry coating agent, for example, the Opadry coating agent of white, blue, yellow, red, green or orange; more preferably, Opadry^{®} White and/or Opadry^{®} Blue.

More preferably, the pharmaceutically acceptable excipients simultaneously comprise the above excipients of (1) - (4) or (1) - (5).

Further, in any of the above technical solutions (pharmaceutical composition), the pharmaceutical composition is a solid formulation or a liquid formulation; preferably, the solid formulation is a tablet, for example a core tablet or a coating tablet.

Further, in any of the above technical solutions (pharmaceutical composition), the pharmaceutical composition is an oral formulation.

Further, in any of the above technical solutions (pharmaceutical composition), under the following measuring conditions, the pharmaceutical composition has a pH value of ≤4, preferably, a pH value of 1 - 4, for example, 1, 1.5, 2, 2.5, 3, 3.1, 3.2, 3.3, 3.4, 3.5, 3.6, 3.7, 3.8, 3.9 or 4, and the like;

When the pharmaceutical composition is a solid formulation, the measuring conditions comprise: a dispersion with a concentration of 20%-30% (that is, 0.2g/ml ~ 0.3g/ml, such as 0.2g/ml) is obtained by mixing the pharmaceutical composition with water (pH = 6 ± 0.1), then the pH value of the dispersion is measured;

When the pharmaceutical composition is a liquid formulation, the measuring conditions comprise: a solvent of the liquid formulation is water (pH = 6 ± 0.1), and the concentration of the liquid formulation is 20%-30% (g/ml), then the pH value of the liquid formulation is measured.

The measuring conditions for the above pH value are not a limitation to the pharmaceutical composition. As long as the above measuring conditions are used for measurement and the obtained pH values are the same as those recited above or within the error ranges, they fall within the protection scope of the present disclosure.

Further, in any of the above technical solutions (pharmaceutical composition), the pharmaceutically acceptable acid is a solid acid or a liquid acid;
preferably, the solid acid is selected from the group consisting of one or more of tartaric acid, succinic acid, maleic acid, fumaric acid, citric acid, malic acid, ascorbic acid, benzenesulfonic acid, oxalic acid, triphenylacetic acid, 1-hydroxyl-2-naphthalenemethyl acid, and 3-hydroxyl-2-naphthalenemethyl acid; and the liquid acid is selected from the group consisting of one or more of hydrochloric acid, sulfuric acid, phosphoric acid, hydrofluoric acid, hydrobromic acid, acetic acid, trifluoroacetic acid, propanoic acid, methanesulfonic acid, and trifluoromethanesulfonic acid; more preferably, the pharmaceutically acceptable acid is a solid acid; still further preferably, the pharmaceutically acceptable acid is tartaric acid (L(+)tartaric acid or D (-) tartaric acid).

Further, in any of the above technical solutions (pharmaceutical composition), the pharmaceutical composition comprises the following components in parts by weight: 1.71 parts of secondary amine compounds; 100-250 parts of diluent; 2-10 parts of disintegrant; 0-5 parts of glidant; 0-5 parts of lubricant; and 0.01-15 parts of pharmaceutically acceptable acid.

Further, in any of the above technical solutions (pharmaceutical composition), the pharmaceutical composition is composed of the following components in parts by weight: 1.71 parts of secondary amine compounds; 100-250 parts of diluent; 2-10 parts of disintegrant; 0-5 parts of glidant; 0-5 parts of lubricant; and 0.01-15 parts of pharmaceutically acceptable acid.

Further, in any of the above technical solutions (pharmaceutical composition), the pharmaceutical composition comprises the following components in parts by weight: 1.71 parts of secondary amine compounds; 184.47 parts of diluent; 4 parts of disintegrant; 0.6 part of glidant; 5 parts of lubricant; and 1.71-3.42 parts of pharmaceutically acceptable acid.

Further, in any of the above technical solutions (pharmaceutical composition), the pharmaceutical composition is any one of the formulations as follows:
Formulations 1:
   The pharmaceutical composition is composed of the following components in parts by weight: 1.71 parts of varenicline tartrate; 64 parts of anhydrous calcium bicarbonate; 120.47 parts of microcrystalline cellulose; 4 parts of partially pregelatinized maize starch; 0.6 part of colloidal silicon dioxide; 5 parts of stearic acid; and 3.42 parts of L (+) tartaric acid.
Formulations 2:
   The pharmaceutical composition is composed of the following components in parts by weight: 1.71 parts of varenicline tartrate; 64 parts of anhydrous calcium bicarbonate; 120.47 parts of microcrystalline cellulose; 4 parts of partially pregelatinized maize starch; 0.6 part of colloidal silicon dioxide; 5 parts of stearic acid; and 1.71 parts of L (+) tartaric acid.
Formulations 3:
   The pharmaceutical composition is composed of the following components in parts by weight: 1.71 parts of varenicline tartrate; 64 parts of anhydrous calcium bicarbonate; 120.47 parts of microcrystalline cellulose; 4 parts of partially pregelatinized maize starch; 0.6 part of colloidal silicon dioxide; 5 parts of stearic acid; 3.42 parts of L (+) tartaric acid; and 6 parts of Opadry - white (premix of Opadry white) or Opadry-blue (premix of Opadry blue).
Formulations 4:
   The pharmaceutical composition is composed of the following components in parts by weight: 1.71 parts of varenicline tartrate; 64 parts of anhydrous calcium bicarbonate; 120.47 parts of microcrystalline cellulose; 4 parts of partially pregelatinized maize starch; 0.6 part of colloidal silicon dioxide; 5 parts of stearic acid; 1.71 parts of L (+) tartaric acid; and 6 parts of Opadry - white (premix of Opadry white) or Opadry-blue (premix of Opadry blue).

Further, in any of the above technical solutions (pharmaceutical composition), the content of nitrosamine impurities in the pharmaceutical composition is not more than 7.5 ppm (for example, 7.4 ppm, 7.3 ppm, 7.2 ppm, 7.1 ppm, 7.0 ppm, 6.9 ppm, 6.8 ppm, 6.7 ppm, 6.6 ppm, 6.5 ppm, 6.4 ppm, 6.3 ppm, 6.2ppm, 6.1ppm, 6.0ppm, 5.9 ppm, 5.8 ppm, 5.7 ppm, 5.6 ppm, 5.5 ppm, 5.4 ppm, 5.3 ppm, 5.2 ppm, 5.1 ppm, 5.0 ppm, 4.9 ppm, 4.8 ppm, 4.7ppm, 4.6 ppm, 4.5 ppm, 4.4 ppm,4.3 ppm, 4.2 ppm, 4.1 ppm, 4.0 ppm, 3.9 ppm, 3.8 ppm, 3.7 ppm, 3.6 ppm, 3.5 ppm, 3.4 ppm, 3.3 ppm, 3.2 ppm, 3.1 ppm, 3.0 ppm, 2.9 ppm, 2.8 ppm, 2.7 ppm, 2.6 ppm, 2.5 ppm, 2.4 ppm, 2.3 ppm, 2.2 ppm, 2.1 ppm or 2.0 ppm, and the like) ; for example, not more than 7.4 ppm, not more than 7.3 ppm, not more than 7.2 ppm, not more than 7.1 ppm, not more than 7.0 ppm, not more than 6.9 ppm, not more than 6.8 ppm, not more than 6.7 ppm, not more than 6.6 ppm, not more than 6.5 ppm, not more than 6.4 ppm, not more than 6.3 ppm, not more than 6.2 ppm, not more than 6.1 ppm, not more than 6.0 ppm, not more than 5.9 ppm, not more than 5.8 ppm, not more than 5.7 ppm, not more than 5.6 ppm, not more than 5.5 ppm, not more than 5.4 ppm, not more than 5.3 ppm, not more than 5.2 ppm, not more than 5.1 ppm, not more than 5.0 ppm, not more than 4.9 ppm, not more than 4.8 ppm, not more than 4.7 ppm, not more than 4.6 ppm, not more than 4.5 ppm, not more than 4.4 ppm, not more than 4.3 ppm, not more than 4.2 ppm, not more than 4.1 ppm, not more than 4.0 ppm, not more than 3.9 ppm, not more than 3.8 ppm, not more than 3.7 ppm, not more than 3.6 ppm, not more than 3.5 ppm, not more than 3.4 ppm, not more than 3.3 ppm, not more than 3.2 ppm, not more than 3.1 ppm, not more than 3.0 ppm, not more than 2.9 ppm, not more than 2.8 ppm, not more than 2.7 ppm, not more than 2.6 ppm, not more than 2.5 ppm, not more than 2.4 ppm, not more than 2.3 ppm, not more than 2.2 ppm, not more than 2.1 ppm or not more than 2.0 ppm.

Further, the nitrosamine impurity is

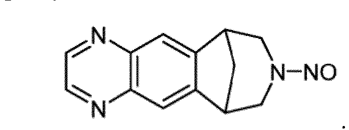

The present disclosure also provides a method for preparing the pharmaceutical composition according to any one of the above, comprising the steps of mixing all of the components and then granulating and tableting, and then optionally comprising a coating step to obtain final product.

Preferably, the method of preparation comprises the steps of screening the colloidal silicon dioxide, anhydrous dibasic calcium phosphate, varenicline tartrate and the pharmaceutically acceptable acid with a 40-mesh sieve, mixing evenly, then adding microcrystalline cellulose, partially pregelatinized maize starch, and intra-granular stearic acid, mixing evenly and granulating, then adding extra-granular stearic acid, mixing evenly and tableting, and then optionally comprises a coating step to obtain final product.

More preferably, the granulating is a dry granulation or a wet granulation; and the tableting is the direct compression process.

The present disclosure also provides the use of the pharmaceutical composition according to any above technical solutions in the preparation of a medicament; and the pharmaceutical composition is used to prevent or treat the following diseases: inflammatory bowel disease, ulcerative colitis, pyoderma gangrenosum, Crohn's disease, irritable bowel syndrome, spastic dystonia, chronic pain, acute pain, celiac sprue, pouchitis, vasoconstriction, anxiety, panic disorder, depression, bipolar disorder, autism, sleep disorders, jet lag, amyotrophic lateral sclerosis, cognitive dysfunction, cognitive impairment caused by alcohol, barbiturates, vitamin deficiencies, recreational drugs, lead, arsenic, or mercury; Alzheimers disease, senile dementia, vascular dementia, Parkinson's disease, multiple sclerosis, AIDS, encephalitis, trauma, hepatic and renal encephalopathy, hypothyroidism, Pick's disease, Korsakoff's syndrome, cognitive impairment caused by frontal dementia or subcortical dementia, hypertension, bulimia, anorexia, obesity, cardiac arrhythmias, gastric acid hypersecretion, ulcers, pheochromocytoma, progressive supramuscular palsy, chemical dependencies on and addictions to nicotine, tobacco products, alcohol, benzodiazepines, barbiturates, opioids or cocaine, headache, migraine, stroke, traumatic brain injury, obsessive-compulsive disorder, psychosis, Huntington's chorea, tardive dyskinesia, hyperkinesia, dyslexia, schizophrenia, multi-infarct dementia, age-related cognitive decline, epilepsy (including petit mal absence epilepsy), attention deficit hyperactivity disorder, or Tourette's Syndrome.

Preferably, the medicament meets one of the following conditions of a-c:
a. The medicament is used to treat the dependencies on, addictions to or deaddictions to nicotine;
b. The medicament is used to treat the dependencies on, addictions to or deaddictions to tobacco products; and
c. The medicament is a smoking cessation medicament.

The other purpose of the present disclosure is providing a new application (use).

The specific technical solution is: a use of pharmaceutically acceptable acid to inhibit or retard the nitrosamineization of the secondary amine compound (nitrosaminization refers to the nitrosamination of a secondary amine structure to generate a nitrosamine structure); wherein the secondary amine compound is varenicline or the pharmaceutically acceptable salt thereof.

The present disclosure also provides a method for inhibiting or retarding the nitrosaminization of secondary amine compound, specifically comprising mixing the secondary amine compound or the composition thereof with pharmaceutically acceptable acids; wherein the secondary amine compound is varenicline or the pharmaceutically acceptable salt thereof.

Further, the method for inhibiting or retarding the nitrosaminization of the secondary amine compound comprises the steps of adding the pharmaceutically acceptable acid in the secondary amine compound or the composition thereof, and mixing evenly.

Further, the composition of the secondary amine compound comprises all of the components of any one of the above pharmaceutical composition, except the pharmaceutically acceptable acid.

Further, the use of the pharmaceutically acceptable acid to inhibit or retard the nitrosaminization of the secondary amine compound and the method for inhibiting or retarding the nitrosaminization of the secondary amine compound independently meet one or more of the conditions Ia-Id as follows (the condition Ia is satisfied separately, the condition Ib is satisfied separately, the condition Ic is satisfied separately, and the like):
Ia. The secondary amine compound and the pharmaceutically acceptable acid are at a molar ratio of 1: (0.01-50) (e.g., at a molar ratio of 1:0.05, 1:0.1, 1:0.5, 1:1, 1:1.5, 1:2, 1:2.5, 1:3, 1:4, 1:5, 1:6, 1:7, 1:8, 1:9, 1:10, 1:15, 1:20, 1:25, 1:30 or 1:40, and the like) ;
Ib. The secondary amine compound is the pharmaceutically acceptable salt of varenicline;
Ic. The pharmaceutically acceptable acid is a solid acid or a liquid acid, and the solid acid is selected from the group consisting of one or more of tartaric acid, succinic acid, maleic acid, fumaric acid, citric acid, malic acid, ascorbic acid, benzenesulfonic acid, oxalic acid, triphenylacetic acid, 1-hydroxyl-2-naphthalenemethyl acid, and 3-hydroxyl-2-naphthalenemethyl acid; and the liquid acid is selected from the group consisting of one or more of hydrochloric acid, sulfuric acid, phosphoric acid, hydrofluoric acid, hydrobromic acid, acetic acid, trifluoroacetic acid, propanoic acid, methanesulfonic acid, and trifluoromethanesulfonic acid;
Id. The addition amount of the pharmaceutically acceptable acid is such that the pH value of mixture X under the following measuring conditions is ≤4; wherein the mixture X comprises the pharmaceutically acceptable acid and the secondary amine compound; preferably, the pH value is 1-4, such as 1, 1.5, 2, 2.5, 3, 3.1, 3.2, 3.3, 3.4, 3.5, 3.6, 3.7, 3.8, 3.9 or 4, and the like; when the mixture X is a solid, the measuring condition comprises: a dispersion with a concentration of 20%-30% (e.g., 20%) is obtained by mixing the mixture X with water (pH = 6 ± 0.1), then the pH value of the dispersion is measured; and when the mixture X is a liquid, the measuring conditions comprise: the solvent of the liquid is water (pH = 6 ± 0.1), and the concentration of the liquid is 20%-30% (e.g., 20%), then the pH value of the liquid is measured.

Preferably, the use of the pharmaceutically acceptable acid to inhibit or retard the nitrosaminization of the secondary amine compound and the method for inhibiting or retarding the nitrosaminization of the secondary amine compound independently meet one or more of the conditions of IIa-IIc as follows (e.g., the condition IIa is satisfied separately, the condition IIb is satisfied separately, the condition IIc is satisfied separately, and the like):
IIa. The secondary amine compound and the pharmaceutically acceptable acid are at a molar ratio of 1: (1-20); further preferably, the secondary amine compound and the pharmaceutically acceptable acid are at a molar ratio of 1: (2-10);
IIb. The secondary amine compound is varenicline hydrochloride or varenicline tartrate;
IIc. The pharmaceutically acceptable acid is a solid acid; further preferably, the pharmaceutically acceptable acid is tartaric acid (L (+) tartaric acid or D (-) tartaric acid).

Further preferably, the use of the pharmaceutically acceptable acid to inhibit or retard the nitrosaminization of the secondary amine compound and the method for inhibiting or retarding the nitrosaminization of the secondary amine compound independently meet the conditions of Ia-Id and IIa-IIc at the same time.

Further, in the use of the pharmaceutically acceptable acid to inhibit or retard the nitrosaminization of the secondary amine compound and the method for inhibiting or retarding the nitrosaminization of the secondary amine compound, the inhibition or the retardation of the nitrosaminization of the secondary amine compound refers to the inhibition or the retardation of the nitrosaminization of the secondary amine compound to generate

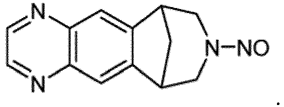

The present disclosure provides a compound of formula P08, the pharmaceutically acceptable salt, solvate thereof and the solvate of the pharmaceutically acceptable salt thereof.

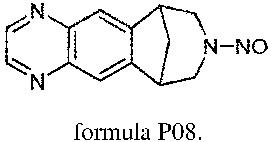

The present disclosure also provides a use of a compound of formula P08 or the pharmaceutically acceptable salt, crystal form, solvate thereof and the solvate of a pharmaceutically acceptable salt thereof as insecticide:

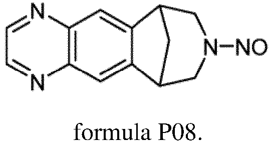

In the use of a compound of formula P08 or the pharmaceutically acceptable salt, crystal form, solvate thereof and the solvate of a pharmaceutically acceptable salt thereof as insecticide, the insecticide is used for controlling Tetranychus cinnabarinus.

The present disclosure also provides a use of a compound of formula P08 as a standard substance, a control substance, or a detection item in impurity studies, quality control or measuring method for active pharmaceutical ingredients or formulation of secondary amine compound; wherein the secondary amine compound is varenicline or a pharmaceutically acceptable salt thereof, and the measuring method is liquid chromatography-tandem mass spectrometry:

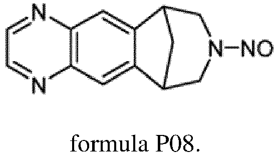

On the basis of conforming to the common sense in the art, the above preferred conditions can be arbitrarily combined to obtain the preferred embodiments of the present disclosure.

The reagents and raw materials used in the present disclosure are commercially available.

The advantageous effects of the present disclosure lie in: the pharmaceutical composition of the present disclosure can effectively inhibit and reduce the generation of nitrosamine impurities, and improve the stability of the secondary amine compound or the composition thereof, thus control the content of genotoxic nitrosamine impurities at a low level by addition of the pharmaceutically acceptable acid to the secondary amine compound (varenicline or the pharmaceutically acceptable salts thereof). Accordingly, the pharmaceutical composition of the present disclosure meets the safety requirements and the provisions of drug supervision and administration and better protects the safety of drug use of patients.

Obviously, according to the above description of the present disclosure and the common technical knowledge and customary means in the art, other various modifications, replacements or changes can be made, without departing from the basic technical ideas of the present disclosure.

### BRIEF DESCRIPTION OF FIGURES

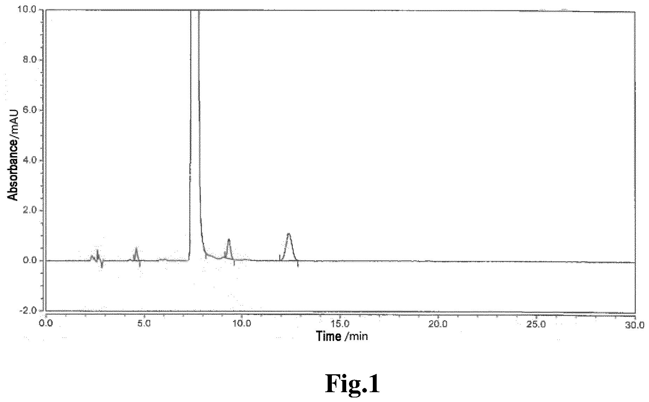

Figure 1 shows the chromatogram of the nitrosamine impurity compound P06 in the compound A detected by high performance liquid chromatography.

The present disclosure will be further illustrated below by means of examples. However, the present disclosure is not limited to the scope of the said examples. For the experimental methods that do not specify specific conditions in the following examples, specific conditions shall be selected in accordance with the conventional methods and conditions, or in accordance with the product specification.

### DETAILED DESCRIPTION OF THE DISCLOSURE

### Example 1

**Table 1: Components and the contents thereof of formulation A (spec 1mg).**

| No. | Component | | mg/tablet | Supplier |
|---|---|---|---|---|
| Intra-granular | | | | |
| 1 | Varenicline Tartrate | | 1.71 | -- |
| 2 | Anhydrous dibasic calcium phosphate | Part I | 20 | Budenheim |
| | | Part II | 44 | Budenheim |
| 3 | Colloidal silicon dioxide | Part I | 0.6 | Evonik |
| | | Part II | 0.8 | Evonik |
| 4 | Microcrystalline Cellulose | | 120.47 | JRS |
| 5 | Partially Pregelatinized Maize Starch (Starch 1500) | 4 | Colorcon | |
| 6 | L (+) Tartaric acid | 3.42 | Merck | |
| 7 | Stearic acid | 1 | BASF | |
| Extra-granular | | | | |
| 8 | Stearic acid | 4 | BASF | |
| **Core Tablet weight** | | **200** | | |
| Film coating agent | | | | |
| 1 | Opadry-White (premix of Opadry White) | 6 | Colorcon | |
| 2 | Purified Water | 54 | | |
| **Total tablet weight** | | **206** | | |

Wherein,
1.71 mg/tablet of the varenicline Tartrate can be converted into 1 mg/tablet of varenicline, i.e., each tablet contains 1mg active ingredient (varenicline), which can also be expressed as: 1mg core tablet (or 1mg coating tablet, and the like). 0.5mg tablet is that the content of each component is reduced to half on the basis of the 1mg tablet. In the above formulation, microcrystalline cellulose and anhydrous dibasic calcium phosphate were used as a diluent, partially pregelatinized maize starch was used as a disintegrant, colloidal silicon dioxide was used as a glidant, stearic acid was used as a lubricant, L (+) Tartaric acid was used as acidity regulator (or pH regulator), and purified water was used as a coating solvent.

The method of preparation (130,000 tablets per batch) comprises the steps as follows:
(1) Screening and mixing
a. The colloidal silicon dioxide (Part I) and anhydrous dibasic calcium phosphate (Part I) were screened with a 40-mesh sieve, screened by a discharger (diameter: 1.2mm, speed: 400±10 r/min), and then transferred into a stirrer (speed: 15r/min) to stir for 2 min.
b. The varenicline tartrate was screened with a 40-mesh sieve, screened by a discharger (diameter: 1.2mm, speed: 800±10 r/min), and then transferred into the above stirrer to stir for further 10 min.
c. The colloidal silicon dioxide (Part II), anhydrous dibasic calcium phosphate (Part II), and L (+) Tartaric acid were screened with a 40-mesh sieve, screened by a discharger (diameter: 1.2mm, speed: 400±10 r/min), and then transferred into the above stirrer to stir for further 10 min.
d. The microcrystalline cellulose, partially pregelatinized maize starch and stearic acid (intra-granular) were screened by a discharger (diameter: 1.2mm, speed: 100±5 r/min), and transferred into the above stirrer to stir for further 10 min.

(2) Dry granulating
The above mixture was granulated by a Dry Granulator LGS120, the specific parameters were as follows: screen: 1.0mm; feeding speed: 20-30rpm; roller speed: 10±2 rpm; roller gap: 0.5-3.0mm; oil pressure: 60-70bar; and rotating speed: 100-140rpm.
(3) Final mixing
The granules after granulation and the stearic acid (extra-granular) were added into a stirrer to stir for 10min to give the final mixture.
(4) Tableting (the preparation of 1mg core tablet A)
The above final mixture was tableted with the specific parameters shown in the Table 2.

**Table 2: The main process parameters of tableting.**

| | | |
|---|---|---|
| Parameter | Spec 1mg | Spec 0.5mg |
| Mold | 10×5 mm oval-shaped | 8×4 mm oval-shaped |
| Speed | 52 rpm | 40 rpm |
| Hardness | 120-180N | 70-130N |
| Main pressure scale value | 9950-10750 | 10050-10850 |
| Fill scale value | 3500-4500 | 3000-4000 |

(5) Coating (the preparation of 1mg white coating tablet A)
The Opadry White (premix of Opadry White) was added into the purified water in proportion, mixed evenly to give a coating dispersion. The tablet (core tablet) was coated with a coating machine with the specific parameters as follows: pan speed: 3-12r/min; inlet/outlet air rotating speed: 1200/1600 rpm; inlet air temperature: 60°C; starting spray gun pressure: 2.5±0.5 bar; atomization pressure: 2.0±0.5 bar; latex tube diameter: 4.00-5.00mm; and peristaltic pump rotating speed: 20-50r/min.
The coating agent was replaced with the Opadry Blue (premix of Opadry Blue), and other conditions remained unchanged to give 1mg blue coating tablet A.

(6) Packaging with the aluminum-plastic.

### Example 2

Formulation B (spec 1mg): The content of the L (+) tartaric acid was changed to 1.71 mg/tablet, and other conditions remained unchanged. According to the same process of preparation as in the example 1, the 1mg core tablet B, 1mg white coating tablet B, and 1mg blue coating tablet B were prepared.

### Example 3

Formulation Y (spec 1mg): The L (+) tartaric acid was not added, and other conditions remained unchanged. According to the same process of preparation as in the example 1, 1mg core tablet Y, 1mg white coating tablet Y, and 1mg blue coating tablet Y were prepared.

### Example 4

The prepared varenicline tartrate tablets were dispersed into purified water (after measuring, pH of the purified water = 6.01) to give a dispersion with a concentration of 20% (g/ml) (indicating that 100ml of solution or dispersion contains 20g varenicline tartrate tablets). After ultrasound for 5min, the pH of the dispersion was measured. The content of the nitrosamine impurity P08 in the varenicline tartrate tablets were measured by HPLC Q-Exactive-MS/MS (Vanquish HPLC Q-Exactive-MS/MS).

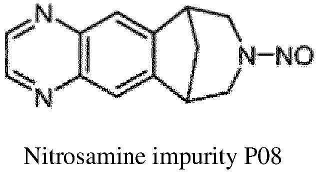

The measurements are shown in the Tables 3-5.

**Table 3: The measurement results of pH value and the content of nitrosamine impurity P08 (Core Tablet).**

| P08 in API (ppm) | Measurement item | | Initial | | Measurement at high temperature (60°C, 5h) |
|---|---|---|---|---|---|
| | | | pH value | P08 (ppm) | P08 (ppm) |
| 0.511 | Example 3 | 1mg core tablet Y | -- | 1.35 | 16.04 |
| | | | Adjusted with NaOH pH=7.0 | 231.69* | 6122.18^{#} |
| | | | Adjusted with NaOH pH=9.73 | 4478.08* | 9060.5^{#} |
| | Example 1 | 1mg core tablet A | -- | 0.82 | 0.88 |
| | Example 2 | 1mg core tablet B | -- | 0.95 | 1.07 |

**Table 4: The measurement results of pH value and the content of nitrosamine impurity P08 (White Coating Tablet).**

| P08 in API (ppm) | Measurement item | | Initial | | Measurement at high temperature (60°C, 5h) |
|---|---|---|---|---|---|
| | | | pH valu e | P08 (ppm) | P08 (ppm) |
| 0.511 | Example 3 | 1mg white coating tablet Y | 5.56 | 1.06 | 11.16 |
| | Example 1 | 1mg white coating tablet A | 3.44 | 0.76 | 0.81 |
| | Example 2 | 1mg white coating tablet B | 3.65 | 0.92 | 1.05 |

**Table 5: The measurement results of pH and the content of nitrosamine impurity P08 (Blue Coating Tablet).**

| P08 in API (ppm) | Measurement item | | Initial | | Measurement at high temperature (60°C, 5h) |
|---|---|---|---|---|---|
| | | | pH value | P08 (ppm) | P08 (ppm) |
| 0.511 | Example 3 | 1mg blue coating tablet Y | -- | 1.46 | 13.55 |
| | Example 1 | 1mg blue coating tablet A | -- | 0.89 | 1.02 |
| | Example 2 | 1mg blue coating tablet B | -- | 0.97 | 1.13 |

Wherein,
"API" (Active Pharmaceutical Ingredient) refers to the API of varenicline tartrate.
"Initial" refers to the immediate measurement after the preparation of the varenicline tartrate tablet.
"Measurement at high-temperature" refers to the measurement after keeping at 60°C of the prepared varenicline tartrate tablet for a period of time (see "Chinese Pharmacopoeia").
Symbol * refers to the liquid sample after adjusting the pH value, and the liquid sample is tested.
Symbol # refers to the liquid sample after adjusting the pH value which is subjected to a high temperature test, and then the liquid sample is tested.

### Example 5

Methodologically validated HPLC Q-Exactive-MS/MS (Vanquish HPLC Q-Exactive-MS/MS) was used for the qualitative and/or quantitative determination of nitrosamine impurity P08 in varenicline tartrate tablets. The measurement results are shown in Example 4.

The chromatographic and the mass spectrum conditions of HPLC Q-Exactive-MS/MS are shown in Table 6.

**Table 6: The chromatographic and the mass spectrum conditions of HPLC Q-Exactive-MS/MS.**

| **Parameter** | **Specific configuration** | | | |
|---|---|---|---|---|
| Instrument | Vanquish HPLC Q-Exactive-MS/MS | | | |
| Chromatographic conditions | Chromatographic column | ChromCore Biphenyl (2.1mm×100mm, 3µm), or equivalent column | | |
| | Column temperature | 25°C | | |
| | Flow rate | 0.40ml/min | | |
| | Injection volume | 20µl | | |
| | Mobile phase A | Formic acid-purified water (0.05:100) | | |
| | Mobile phase B | Acetonitrile | | |
| | Gradient elution | Time(min) | Mobile phase A (%) | Mobile phase B (%) |
| | | 0 | 95 | 5 |
| | | 2 | 95 | 5 |
| | | 13 | 3 | 70 |
| | | 15 | 30 | 70 |
| | | '6 | 95 | 5 |
| | | 20 | 95 | 5 |
| Mass spectrometer conditions | Spray voltage | 3.9 kV | | |
| | Sheath gas flow rate | 35 arb | | |
| | Aux gas flow rate | 10 arb | | |
| | Capillary temperature | 320°C | | |
| | Aux gas heter Temperature | 320°C | | |
| | Scan type | SIM (single ion monitoring) | | |
| | Scan range | 240.3-241.9 m/z | | |
| | Polarity | Positive | | |

### 1. Preparation of solutions

Control stock solution: 7.5 mg of the nitrosamine compound P08 (purity≥98%) was weighed into a 100ml volumetric flask, dissolved and diluted with acetonitrile to volume (volumetric solution 1A). 1.0mL of the volumetric solution 1A was added into a 100ml volumetric flask, and diluted with acetonitrile to volume (volumetric solution 1B). 1.0mL of the volumetric solution 1B was added into a 20ml volumetric flask, and diluted with acetonitrile to volume to obtain the control stock solution.

Control solution: 1.0mL of the control stock solution was added into a 10ml volumetric flask, and diluted with acetonitrile to volume to obtain the control solution.

Sample solution: 10 varenicline tartrate tablets were placed in a 10ml volumetric flask, added with 3mL of purified water and 3mL of acetonitrile, sonicated for 30s, then diluted with acetonitrile to volume and mixed evenly.

Sample spiked solution: 10 varenicline tartrate tablets were placed in a 10ml volumetric flask, added with 3mL of purified water and 3mL of acetonitrile, sonicated for 30s, and added with 1mL of the control stock solution, and then diluted with acetonitrile to volume and mixed evenly.

Placebo solution: 1g of placebo (equivalent to a tablet without the active ingredient) was weighed into a 10ml volumetric flask, 3mL of purified water and 3mL of acetonitrile were added, sonicated for 30s, diluted with acetonitrile to volume and mixed evenly.

Placebo spiked solution: 1g of placebo was placed into a 10ml volumetric flask, added with 3mL of purified water and 3mL of acetonitrile, sonicated for 30s, added with 1mL of the control stock solution, and then diluted with acetonitrile to volume and mixed evenly.

Blank: Acetonitrile.

### 2. Specificity:

The blank, control solution, sample solution, placebo solution, sample spiked solution, placebo spiked solution were injected for analysis respectively.

The results showed that the retention times of the target substance of the control solution, sample solution and sample spiked solution were about 7.80 min, and there was no obvious interference at the peak position of the target substance, which met the requirement of separation and meant a good specificity.

### 3. Stability of solutions:

The control solution and the sample solution were placed at room temperature, then injected for analysis at intervals of 0, 2, 4, 6, 8, 10, 12 hours, and the peak area ratio of each time point to the zero point (i.e., peak area change rate) was calculated.

The results showed that the peak area change rate of the nitrosamine compound P08 in the control solution within 12 hours is <12%, and the peak area change rate of the nitrosamine compound P08 in the sample solution within 12 hours is <3.5%.

### 4. System suitability:

The control solution was injected and analyzed 5 times, and the RSD of the peak area of the target compound was calculated as 1.41%, indicating that the system meant a good suitability.

### 5. Repeatability:

6 sample solutions were prepared in parallel and injected for analysis respectively. The results showed that the RSD of the content of impurity compound P08 in the 6 sample solutions was 3.49%, which met the acceptance criteria and meant a good repeatability.

### 6. Intermediate precision:

6 sample solutions were weighed by two analysts respectively at different times, injected for analysis, and the average and the RSD were calculated. The results showed the RSD=5.93%.

### 7. Linearity:

The control solution with concentrations of 0.015ng/ml, 0.38ng/ml, 1.88ng/ml, 3.77ng/ml, 5.65ng/ml, 18.83ng/ml, 37.66ng/ml and 753.2ng/ml were prepared respectively and injected for analysis: The linear regression was carried out, wherein the concentration of the target substance (X) was taken as the horizontal coordinate, and the peak area (Y) was taken as the ordinate. The linear equation and linear correlation coefficient r were obtained by calculating.

The results showed that the linear equation is Y=462785X+1000000, and the linear correlation coefficient r=0.9999, which illustrates that the linearity is good in the concentration range of 0.0 15ng/ml-753 .2ng/ml.

### 8. Accuracy

The results showed that the recovery rate of sample spiked solution was between 85% to 95%, and the RSD was 2.03%, which met the acceptance criteria and meant a good accuracy.

### 9. Limit of detection (LOD):

LOD=13.2 ppb, the average S/N (signal-to-noise ratio) of the target peak was 75.94, which met the requirement of greater than 3, showing that the method was sensitive.

### 10. Limit of quantitation (LOQ):

LOQ=17.6 ppb, the average S/N (signal-to-noise ratio) of the target peak was 103.35, which met the requirement of greater than 10, showing that the method was sensitive.

### 11. Durability:

The results showed that the method had a good durability when the column temperature was varied by ±2°C.

The content of the nitrosamine impurity compound P08 was calculated with the following equation: C (ppm) = the concentration (ng/ml) of P08 in the sample solution / the concentration (mg/ml) of varenicline tartrate in the sample solution.

### Example 6

The preparation of the nitrosamine compound P08:

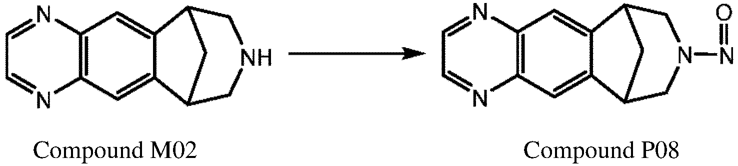

4.0g of the compound M02 (varenicline) and 45mL of tetrahydrofuran (THF) were added into a 100mL three-necked flask and stirred to dissolve. Then, 8.2mL of aqueous solution of NaNO₂ (concentration of 0.4g/mL) and 2.9g of acetic acid were added and heated up to 52°C. When the reaction was completed by monitoring via TLC, the solid was precipitated, filtered and vacuum dried to give the nitrosamine compound P08 with a purity of 98.87% (area normalization method).

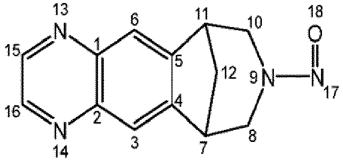

The ¹H-NMR, ¹³C-NMR and IR spectrums of the nitrosamine compound P08 are shown in the following Table 7-9 respectively; M+H⁺=241.20; UV: the maximum absorption wavelength in the solution of acetonitrile is 204.40 nm.

**Table 7: The ¹H-NMR spectrum of the nitrosamine compound P08.**

| Chemical shift | Multiplicity | Integration | Attribution |
|---|---|---|---|
| 8.83 | dd | 2H | H-15, H-16 |
| 8.04 | s | 1H | H-3 |
| 7.90 | s | 1H | H-6 |
| 4.97 | d | 1H | H-8 |
| 4.83 | d | 1H | H-10 |
| 4.36 | d | 1H | H-7 |
| 3.79 | s | 1H | H-11 |
| 3.64 | s | 1H | H-8 |
| 3.20 | d | 1H | H-10 |
| 2.57 | m | 1H | H-12 |
| 2.39 | d | 1H | H-12 |

**Table 8: The ¹³C-NMR spectrum of the nitrosamine compound P08.**

| Chemical shift | Attribution |
|---|---|
| 148.38 | C-16 |
| 148.01 | C-15 |
| 144.27 | C-1, C-2 |
| 142.87 | C-4 |
| 142.85 | C-5 |
| 122.35 | C-3 |
| 122.04 | C-6 |
| 56.28 | C-8 |
| 47.01 | C-10 |
| 40.86 | C-7 |
| 40.23 | C-11 |
| 39.29 | C-12 |

**Table 9: The IR spectrum of the nitrosamine compound P08.**

| absorption wavenumber (cm⁻¹) | Attribution |
|---|---|
| 3048.8 | Ar-H |
| 2961.4, 2931.8 | saturated C-H stretching vibration |
| 1925.2 | benzene ring C=C stretching vibration |
| 1575.2 | N=O stretching vibration |
| 885.2 | benzene ring C-H bending vibration |

### Example 7

Methodologically validated UPLC-MS/MS was used in the qualitative and/or quantitative detection of the nitrosamine impurity (compound P08) in the API of varenicline tartrate.

The chromatographic and mass spectrometer conditions of the UPLC-MS/MS were shown in Table 10.

**Table 10: The chromatographic and mass spectrometer conditions of the UPLC-MS/MS.**

| Parameter | | Specific configuration | | |
|---|---|---|---|---|
| Instrument | | UPLC-MS/MS (ACQuity I CLASS & TRIPLE QUAD 5500+) | | |
| chromatographic conditions | Chromatographic column | ACQUITY UPLC BEH C18 (2.1mm×100mm, 1.7µm) | | |
| | Column temperature | 30°C | | |
| | Flow rate | 0.40ml/min | | |
| | Injection tray temp | 15°C | | |
| | Injection volume | 2µl | | |
| | Mobile phase A | Formic acid-purified water (0.1:100) | | |
| | Mobile phase B | Acetonitrile | | |
| | Gradient elution | Time(min) | Mobile phase A (%) | Mobile phase B (%) |
| | | 0 | 85 | 15 |
| | | 1.5 | 85 | 15 |
| | | 4 | 5 | 95 |
| | | 5 | 5 | 95 |
| | | 6 | 85 | 15 |
| Time event | | 0 min; 2.4 min | | |
| mass spectrometer conditions | Ion Source type | ESI | | |
| | Ion Source model | Positive | | |
| | Scan type | Multi Reaction Monitoring (MRM) | | |
| | Damping gas | 9psi | | |
| | Curtain gas | 30psi | | |
| | IonSpray voltage | 5500V | | |
| | Temperature | 500°C | | |
| | Spray gas | 50psi | | |
| | Auxiliary heat gas | 50psi | | |

### 1. The preparation of solution:

Diluent: purified water.

Control stock solution: 19.970mg nitrosamine compound P08 (purity ≥ 98%) was weighed into a lOmL volumetric flask, added with 8ml of acetonitrile, then dissolved and diluted to volume with the diluent, and mixed evenly.

Control mother solution: 37.5 µl of the control stock solution was measured into a lOmL volumetric flask, diluted to volume with the diluent, and mixed evenly.

### 2. System suitability:

System suitability solution (the control solution of 100% limit level): 100.0µl of the control mother solution was measured into a lOmL volumetric flask, diluted to volume with the diluent, and mixed evenly.

6 needles of the system suitability solution were injected continuously, the RSD of the peak area of the target compound in the 6 needles of the system suitability solution was calculated as 4%, indicating that the system had a good suitability.

### 3. Linearity

Standard curve solution: 50.0µl, 100.0µl, 150.0µl, and 200.0µl of the control mother solution were measured into four of lOmL volumetric flasks respectively, diluted to volume with the diluent and mixed evenly to obtain the linear solutions L-3, L-4, L-5, and L-6. 800µl of the linear solution L-3 was measured into a lOmL volumetric flask, diluted to volume with the diluent, and mixed evenly to obtain the solution L-2. 1000µl of the solution L-2 was measured into a lOmL volumetric flask, diluted to volume with the diluent, and mixed evenly to obtain the solution L-1.

The standard curve solutions were injected respectively for analysis. The linear regression was carried out, wherein the concentration of the target substance (X) was taken as the horizontal coordinate (unit: ng/ml), and the peak area (Y) was taken as the ordinate. The linear equation and linear correlation coefficient r were obtained by calculating. The results showed that the linear equation is Y=7370X-12900, and the linear correlation coefficient r=0.999, which illustrates that the linearity is good in the concentration range of 0.2951ng/ml-147.6ng/ml (i.e., 0.02951ppm-14.76ppm).

### 4. Limit of detection (LOD):

LOD solution: 5000µl of the linear solution L-1 was measured into a lOmL volumetric flask, diluted to volume with the diluent, and mixed evenly to obtain the LOD solution.

2 needles of the LOD solution were continuously injected, the S/N (signal-to-noise ratio) of the target peak was 11 and 8 respectively, which met the requirement of not less than 3. LOD=0.01476 ppm.

### 5. Limit of quantitation (LOQ):

6 needles of the LOQ (solution L-1) solution were continuously injected, the S/N (signal-to-noise ratio) of the target peak was 23, 24, 17, 12, 24, and 14 respectively, which met the requirement of not less than 10. LOQ=0.02951ppm.

### 6. Accuracy:

Sample solution: About 100mg of sample (e.g., purchased or synthesized varenicline tartrate API) was weighed into a lOmL volumetric flask, diluted with the diluent to volume, and mixed evenly. 2 sample solutions were prepared in parallel.

Accuracy solution: About 100mg of sample was weighed into a lOmL volumetric flask, added with the control mother solution of 50.0 µl (50% of limit level), 100.0 µl (100% of limit level), and 150.0 µl (150% of limit level) respectively, and then diluted with the diluent to the scale, and mixed evenly. 3 accuracy solutions with every limit level were prepared in parallel.

The results showed that the recovery rates of accuracy solutions of 50% limit level were between 95% to 102%, RSD was 4%; the recovery rates of accuracy solutions of 100% limit level were between 90% to 103%, and RSD was 7%; the recovery rates of accuracy solutions of 150% limit level were between 98% to 100%, and RSD was 1%, which met the acceptance criteria and meant a good accuracy.

### 7. Repeatability:

About 100mg of sample was weighed into a 10ml volumetric flask, added with 100µl of the control mother solution, diluted with the diluent to the scale, and mixed evenly. 6 solutions were prepared in parallel.

The results showed that the RSD of the content of the nitrosamine compound P08 in the 6 repetitive solutions was 6%, which met the acceptance criteria and meant a good repeatability.

### 8. Specificity:

The blank solution (diluent), control solution of 100% limit level, sample solution (same as solutions used in the "repeatability"), and accuracy solution of 100% limit level were injected for analysis respectively.

The results showed that the retention times of the target substance in the sample solution, control solution of 100% limit level, and accuracy solution of 100% limit level were about 3.47 min, and there was no obvious interference at the peak position of the target substance, which met the requirement of separation and meant a good specificity.

### 9. Intermediate precision:

Intermediate precision solution: About 100mg of sample was weighed into a 10ml volumetric flask, added with 100µl of the control mother solution, diluted with the diluent to the scale, and mixed evenly. 6 solutions were prepared in parallel. The 6 solutions in "7. Repeatability" were also used.

The RSDs of the 6 intermediate precision solutions and the 6 repeatability solutions (i.e., a total of 12 solutions) were calculated as 9%, which met the requirement of not more than 15%, and met the acceptance criteria and meant a good intermediate precision.

### 10. Stability of solution:

The accuracy solution of 100% limit level was placed at 15°C, then injected for analysis at intervals, and the concentration ratio of each time point to the zero point was calculated. The ratio was between 96% to 113% in 21h.

### 11. Durability

The concentrations of the target substance in the accuracy solution of 100% limit level were measured respectively, when the column temperature was varied by ±2°C, the initial rate of the organic phase was varied by ±1%, and the change of flow rate was varied by ±0.01ml/min. 2 needles of solutions were injected under each condition, and the results showed that the RSD of concentration of the target substance in the 6 solutions with the same varied parameter were between 4% to 6%, which illustrated the durability of the method was good.

The content of the nitrosamine impurity compound P08 was calculated with the following equation. The detection was carried out twice, and the average was used: The content of P08: C (ppm) = the concentration (ng/ml) of P08 in the sample solution / [m (mg) / V (ml)].
m: The mass of sample (mg);
V: The diluent volume of sample (ml).

### Example 8

Whether nitrosamine impurity compound P08 is genotoxic (capable of mutagenicity or carcinogenesis) was detected.

### 1. Materials and methods

### 1.1 Experimental strains

Salmonella Typhimurium TA97a, TA98, TA100, TA102, and TA1535 from MOLT OX, USA, were purchased from Shanghai Bioplus Biotech. Co., Ltd. The test was carried out using Salmonella typhimurium strains that were identified as meeting the requirements.

### 1.2 Metabolically activated system

Rat liver S₉ from Shanghai Bioplus Biotech. Co., Ltd.

### 1.3 Test substance

Name: Nitrosamine impurity compound P08 as white powder.

### 1.4 The main instrument and the regents

X SR-204 electronic balance (Mettler, Switzerland); high pressure steam sterilizer (SANYO Company, Japan); insulated thermostatic incubator (Shanghai Yiheng Technology Co., Ltd.); Stuart temperature controlled shaker incubator (Stuart, UK); and BHC-1300 II A2 Biosafety Cabinet (Suzhou Purification Equipment Group Suzhou Antai Airtech Co., Ltd.).

Glucose 6-phosphate from J&K Scientific; coenzyme II from Sinopharm Chemical Reagent Co., Ltd.; nutrient broth from Beijing Landbrige Technology Co.,Ltd.; technical agar powder from Guangdong Huankai Microbial Sci.&Tech. Co.,Ltd.; sterile water for injection from Cisen Pharmaceutical Co., Ltd.; dexon from CHEMSERVICE; methyl methanesulfonate from J&K Scientific; 2-aminofluorene from Shanghai Jingchun Reagent Co.,Ltd.; 1,8-dihydroxyanthraquinone from SIGMA-ALDRICH, USA; cyclophosphamide from SIGMA-ALDRICH, USA; and sodium azide from RIEDEL-SEELIE.

### 1.5: The method of test (Ames test with plate incorporation method)

The experiments were performed using the plate incorporation method.

### 1.5.1 Dose selection and test substance preparation

Dose selection: The experiments were performed using 5mg, 2.5mg, 1.25mg, 0.625mg, 0.3125mg, and 0.15625mg per dish.

Test substance preparation: nitrosamine impurity compound P08 was prepared to a solution of 50mg/ml with dimethyl-sulfoxide, and diluted with dimethyl-sulfoxide to give the solutions of 25mg/ml, 12.5mg/ml, 6.25mg/ml, 3.125mg/ml, and 1.5625mg/ml successively.

### 1.5.2 Test methods

0.1ml of frozen bacterial solutions TA97a, TA98, TA100, TA102 and TA1535 were inoculated into the 10ml nutrient broth mediums respectively, and cultured at 37°C (100 /min) for 10h with shaking (100 times/min). 2.0ml of top layer medium containing 0.5mmol/L histidine - 0.5mmol/L biotin was dispensed into test tubes, sterilized by 0.103MPa for 20min, and kept in a water bath at 50°C. Then, 0.1ml of the test substance solution, 0.5ml of the S₉ mixture (when metabolic activation was required) and 0.1ml of the bacterial solution were added into each tube successively, mixed sufficiently, and poured into the bottom layer agar plates quickly. Then, the plates were turned to distribute even. After being placed horizontally for condensation and solidification, the plates were inverted and incubated in an incubator at 37 °C for 48h, and the number of reverse mutation colonies per dish was counted. Three parallel plates of each dose were made, containing the spontaneous reverse mutation group, the solvent control group and the positive control group.

### 1.6 Test data statistics

The data were expressed as means ± standards deviation (x̅±s), and SPSS 19.0 was used for calculation of data analysis.

### 1.7 Result Determination

If the number of reverse mutation colonies of the test substance is twice or more than those of the solvent control, and presents in a dose-response relationship, then the test substance is determined to be mutagenic positive. If the test substance has a positive reaction and has repeatability under any dose condition, then the test substance is determined to be mutagenic positive. If the test substance tested by the above test strain is negative both with and without S₉, then the test substance is determined to be mutagenic negative.

### 2. Test results

The test results are shown in the Table 11.

**Table 11: Ames test results (x̅±s).**

| Group | Dose µg/ dish | TA97a | | TA98 | | TA100 | |
|---|---|---|---|---|---|---|---|
| | | +S₉ | -S₉ | +S₉ | -S₉ | +S₉ | -S₉ |
| Test substance | 5000 | 310±19 | 159±5 | 53±6 | 32±2 | 378±18 | 206±25 |
| | 2500 | 297±8 | 165±10 | 59±4 | 31±3 | 364±19 | 193±25 |
| | 1250 | 279±5 | 160±15 | 41±6 | 30±3 | 287±13 | 220±10 |
| | 625 | 242±17 | 175±12 | 35±2 | 28±2 | 271±11 | 224±3 |
| | 312.5 | 248±13 | 175±6 | 34±1 | 27±3 | 234±6 | 234±31 |
| | 156.25 | 209±16 | 158±11 | 33±6 | 29±2 | 225±9 | 218±20 |
| solvent control | DMSO | 239±3 | 167±6 | 29±3 | 30±1 | 237±6 | 216±5 |
| Spontaneo us reverse mutation | / | 220±9 | 187±10 | 29±2 | 31±3 | 278±9 | 217±19 |
| positive control | 2-amino fluorene 10µg/ dish | 951±49 | / | 1271±1 05 | / | 1371±27 | / |
| | Dexon 50 µg/ dish | / | 1294±64 | / | 691±43 | / | / |
| | Methyl methanesulfon ate | / | / | / | / | / | 1596±7 1 |
| 1 µl/ dish | | | | | | | |

| Continued table: Ames test results (x̅+s) | | | | | | | |
|---|---|---|---|---|---|---|---|
| Group | Dose µg/ dish | TA102 | | TA1535 | | | |
| | | +S₉ | -S₉ | +S₉ | -S₉ | | |
| Test substance | 5000 | 320±15 | 211±8 | 46±6 | 20±2 | | |
| | 2500 | 283±11 | 219±11 | 38±5 | 28±2 | | |
| | 1250 | 272±17 | 245±4 | 34±3 | 18±2 | | |
| | 625 | 296±21 | 236±20 | 35±7 | 42±4 | | |
| | 312.5 | 253±24 | 237±6 | 20±3 | 36±1 | | |
| | 156.25 | 249±13 | 208±13 | 21±0 | 23±2 | | |
| Solvent control | DMSO | 283±4 | 252±11 | 16±2 | 19±2 | | |
| Spontaneous reverse mutation | / | 340±11 | 247±18 | 14±2 | 19±3 | | |
| Positive control | 1,8-dihydroxyanthraquinone 50 µg/ dish | 921±32 | / | / | / | | |
| | Cyclophosphamide 200µg/ dish | / | / | 171±11 | / | | |
| | Methyl methanesulfonate 1µl/ dish | / | 2418±257 | / | / | | |
| | Sodium azide 1.5µg/ dish | / | / | / | 570±46 | | |

Ames test results showed that under the condition of no S₉, and the dose of the nitrosamine impurity compound P08 was 625µg/ dish, the number of colonies of the TA1535 was more than twice that of the solvent control. Under the condition of adding the S₉ with a dose of 2500 µg/ dish, the number of colonies of the TA98 was more than twice that of the solvent control. When the doses of the S₉ were 5000 µg/ dish, 2500µg/ dish, 1250µg/ dish, and 625µg/ dish, the number of colonies of the TA1535 was more than twice that of the solvent control, and presented in a dose-response relationship. This illustrated that the nitrosamine impurity compound P08 was mutagenic positive on Salmonella typhimurium in the presence and absence of metabolic activation system.

### Example 9

The bactericidal or insecticidal activity of the nitrosamine impurity compound P08 was determined according to the existing technical standards or specifications (e.g., Chinese agricultural industry standard NY/T 1156.4-2006, pesticides guidelines for laboratory bioactivity tests, bactericide, part 4: potted plant test for fungicide control of powdery mildew on wheat; Chinese agricultural industry standard NY/T 1156.5-2006, pesticides guidelines for laboratory bioactivity tests, bactericide, part 5: detached leaf test for fungicide inhibition of Rhizoctonia solani on faba bean; Chinese agricultural industry standard NY/T 1154.5-2006, pesticides guidelines for laboratory bioactivity tests, insecticide, part 6: the immersion test for insecticide activity; Chinese agricultural industry standard NY/T 1154.9-2008, pesticides guidelines for laboratory bioactivity tests, insecticide, part 9: spraying method).

Wherein,
According to the Chinese agricultural industry standard NYT 1154.13-2008, pesticides guidelines for laboratory bioactivity tests, insecticide, part 13: leaf-disc spraying method, the Tetranychus cinnabarinus were inoculated on a leaf-disc of faba bean as the biological test materials. When the tested mite was stabilized on the leaf, the leaf-disc was placed under a Potter spray tower for quantitative spray treatment (2.5mL) (agent: the nitrosamine impurity compound P08, dissolved with DMF into 1% mother solution, then diluted with the distilled water containing 0.1% tween-80 to the test concentration). After the treatment, the leaf-disc was placed in the observation chamber at 24-26°C for culture, and the result was investigated after 24h. The mite was touched with tweezers, and no response is regarded as dead. A blank control without adding the agent was also designed.

The results showed that the nitrosamine impurity compound P08 with a concentration of 500 mg/L induced the 19.6% mortality rate of Tetranychus cinnabarinus (the mortality rate of blank control was 0%), thus showed a certain control efficiency on Tetranychus cinnabarinus. Moreover, with the increase of the concentration of the agent, there was also a certain degree of improvement of the control efficiency.

### Example 10

The preparation of nitrosamine compound P06: 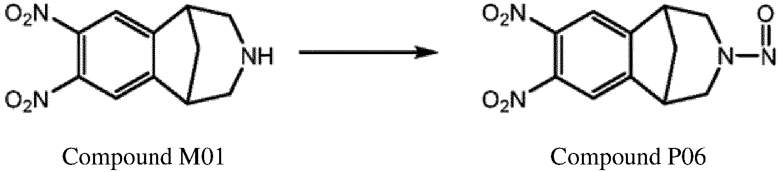 5g of the compound M01 and 52mL of THF were added into a 100mL three-necked flask and stirred to dissolve. Then, lOmL of aqueous solution of NaNO₂ (concentration of 0.4g/mL) and 3g of acetic acid were added and heated up to 50°C. When the reaction was completed by monitoring via TLC or HPLC, the reaction was concentrated, and then added with 50mL of dichloromethane (DCM) and 50mL of saturated brine, stirred to dissolve, left standing to separate into organic phase and aqueous phase. The organic phase was wished with saturated brine, dried over Na₂SO₄, filtered, and concentrated to dry to give the nitrosamine compound P06 with a purity of 93.69% (area normalization method).

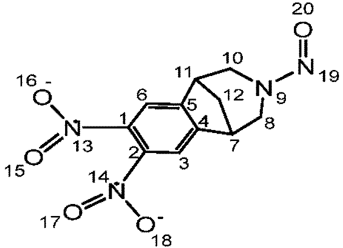

The ¹H-NMR, ¹³C-NMR and IR spectrum of the nitrosamine compound P06, are shown in the following Table 12-14, respectively; M+H⁺=279.10; UV: the maximum absorption wavelength in acetonitrile solution is 223.80 nm.

**Table 12: The ¹H-NMR spectrum of the nitrosamine compound P06.**

| Chemical shift | Multiplicity | Integration | Attribution |
|---|---|---|---|
| 8.24 | s | 1H | H-3 |
| 8.12 | s | 1H | H-6 |
| 4.70 | m | 2H | H-8, H-10 |
| 4.29 | dd | 1H | H-7 |
| 3.69 | m | 1H | H-11 |
| 3.56 | m | 1H | H-8 |
| 3.17 | dd | 1H | H-10 |
| 2.44 | m | 1H | H-12 |
| 2.27 | d | 1H | H-12 |

**Table 13: The ¹³C-NMR spectrum of the nitrosamine compound P06.**

| Chemical shift | Attribution |
|---|---|
| 152.43 | C-4 |
| 151.95 | C-5 |
| 142.23 | C-2 |
| 142.15 | C-1 |
| 121.08 | C-3 |
| 120.63 | C-6 |
| 54.84 | C-8 |
| 46.34 | C-10 |
| 41.29 | C-7 |
| 40.14 | C-11 |
| 39.26 | C-9 |

**Table 14: The IR spectrum of the nitrosamine compound P06.**

| Absorption wavenumber (cm⁻¹) | Attribution |
|---|---|
| 3034.5 | Ar-H |
| 2920.2, 2879.0 | saturated C-H stretching vibration |
| 1667.2 | benzene ring C=C stretching vibration |
| 1546.9, 1428.2, 1344.9 | N=O, N-O stretching vibration |
| 845.2 | benzene ring C-H bending vibration |

### Example 11

The preparation of nitrosamine compound P07:

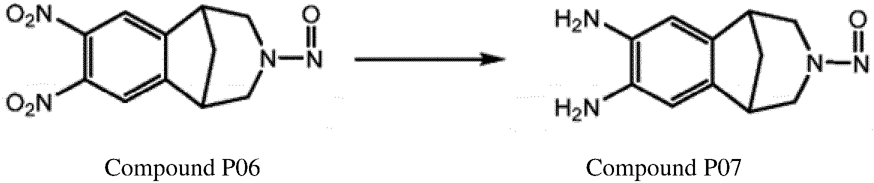

4.0g of the compound P06 and 50mL of dichloromethane (DCM) were added into a 100mL three-necked flask and stirred to dissolve. Then, 1.0g of Pd/C catalyst was added. After replacement with nitrogen, hydrogen gas was continuously injected. The reaction was heated up to 25-30°C to react overnight. When the reaction was completed by monitoring via TLC, the reaction was filtered and concentrated to dry to give the nitrosamine compound P07 with a purity of 96.59% (area normalization method).

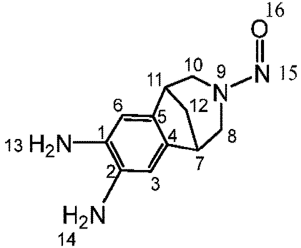

The ¹H-NMR, ¹³C-NMR and IR spectrum of the nitrosamine compound P07, are shown in the following Table 15-17 respectively; M+H⁺=219.20; UV: the maximum absorption wavelength in the solution of acetonitrile : water = 1:1, is 212.00 nm.

**Table 15: The ¹H-NMR spectrum of the nitrosamine compound P07.**

| Chemical shift | Multiplicity | Integration | Attribution |
|---|---|---|---|
| 6.47 | s | 1H | H-3 |
| 6.34 | s | 1H | H-6 |
| 4.44 | d | 1H | H-8 |
| 4.33 | d | 1H | H-10 |
| 4.29 | s | 4H | H-13, H-14 |
| 4.06 | d | 1H | H-7 |
| 3.18 | t | 1H | H-11 |
| 3.03 | m | 1H | H-8 |
| 2.90 | d | 1H | H-10 |
| 2.16 | m | 1H | H-12 |
| 1.97 | d | 1H | H-12 |

**Table 16: The ¹³C-NMR spectrum of the nitrosamine compound P07.**

| Chemical shift | Attribution |
|---|---|
| 134.56 | C-4 |
| 134.52 | C-5 |
| 133.35 | C-2 |
| 132.51 | C-1 |
| 109.97 | C-3 |
| 109.75 | C-6 |
| 56.12 | C-8 |
| 49.07 | C-10 |
| 47.32 | C-7 |
| 41.86 | C-11 |
| 38.62 | C-12 |

**Table 17: The IR spectrum of the nitrosamine compound P07.**

| Absorption wavenumber (cm⁻¹) | Attribution |
|---|---|
| 3432.6 | N-H stretching vibration |
| 3008.2 | Ar-H |
| 2942.2, 2870.9 | saturated C-H stretching vibration |
| 1655.5 | benzene ring C=C stretching vibration |
| 1495.2 | N=O stretching vibration |
| 866.5 | benzene ring C-H bending vibration |

### Example 12

### 1. The preparation of varenicline intermediate:

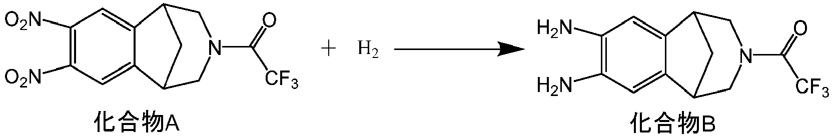

(1). 3kg of the compound A (in order to better ensure product quality, it can generally be required that the content of nitrosamine impurity P06 is ≤ 500ppm, and the measurement value is 485ppm) and 0.35kg of Pd/C (Pd 5%) catalyst were added into a reactor containing 36kg of isopropyl alcohol and 15kg of purified water. Then, the resulting mixture was stirred to react at 25-35°C with injected hydrogen. The reaction was stopped when the residue of the compound A in the reaction solution was ≤0.5% which was monitored via HPLC, and the Pd/C catalyst was removed by filtration with diatomite to obtain the reaction solution containing compound B. 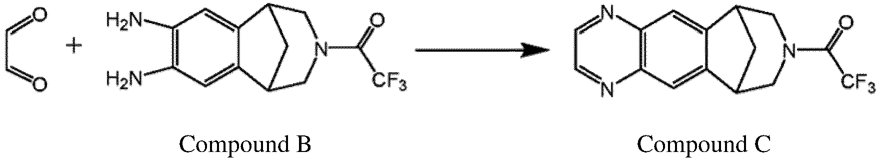
(2). Under protection of inert gas (nitrogen), to the solution obtained from the step (1) was added 75g NaHCO₃. And then the resulting solution was added into the aqueous solution of glyoxal (0.56kg glyoxal and 6.5kg water) slowly, stirred to react at 20-30°C. The reaction was stopped when the residue of the compound B in the reaction was ≤0.5% which was monitored via HPLC. The reaction was subjected to distillation under a reduced pressure (vacuum degree ≤0.09MPa, 40-50°C), until almost no fractions were produced. 90kg of purified water was added into the reactor, and the temperature in the reactor was controlled at 20-30°C for 2 h. Then, the reaction was centrifuged and dried (45-50°C) to give the compound C.

### 2. The preparation of the varenicline tartrate:

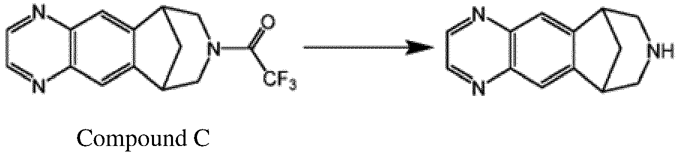

(a). Under protection of inert gas (nitrogen), 20kg of purified water, 0.81kg of NaOH, 8kg of CH₂Cl₂ and 2kg of compound C (prepared from the prior step) were added into a reactor. The mixture was reacted at 20-35°C. The reaction was stopped when the residue of the compound C in the reaction was ≤0.5% which was detected by HPLC. Then, 18.5kg of CHzClz was added into the reactor. The resulting mixture was stirred and then left standing to separate into organic phase and aqueous phase. The aqueous phase was extracted with CH₂Cl₂ (15kg× 2), and then the organic phase was combined, dried over Na₂SO₄ (5kg), and filtered. The filtrate was subjected to distillation under a reduced pressure (vacuum degree ≤0.09MPa, 40-50°C), until almost no fractions were produced. Anhydrous ethanol (10kg) was added, and the mixture was subjected to distillation under a reduced pressure (vacuum degree ≤0.09MPa, 40-50°C) until almost no fractions were produced to obtain the mother solution containing varenicline. 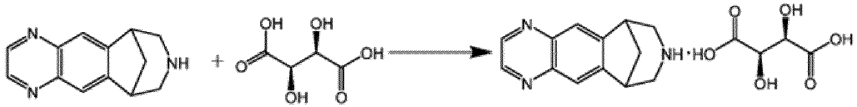
(b). To the mother solution containing the varenicline obtained from the step (a) were added 30kg of anhydrous ethanol and 1.16kg of L (+) Tartaric acid slowly. The solution was stirred to react at 20-30°C for 16h. After the reaction was stopped, the resulting reaction was centrifuged and dried (70-75°C) to give the varenicline tartrate.

According to the trituration solvent 8ml of every gram of varenicline tartrate, the trituration solvent was added the varenicline tartrate obtained from the prior step. The mixture was triturated at 30-40°C for 1.5h, and then centrifuged, and dried. The content of nitrosamine impurity compound P08 in the varenicline tartrate which was triturated with ethyl acetate was detected as 3.04 ppm, and the nitrosamine impurity compounds P06 and P07 were not detected (less than the LOD).

### Examples 13-15

By further controlling the content of nitrosamine impurity compound in the initial materials of the present disclosure (e.g., controlling the content of nitrosamine impurity compound P06 in compound A in the range of ≤ 250ppm, ≤200ppm, ≤ 150ppm, ≤ 100ppm or lower), and increasing the number of trituration treatment, the varenicline tartrates with the content of nitrosamine impurity compound P08 of 0.511ppm, 0.205ppm and 0.118ppm were prepared respectively.

If the content of nitrosamine impurity compound P06 in compound A is too high, the content of P06 could be reduced by the trituration treatment process as follows: For example, 3.3 kg compound A (722 ppm of nitrosamine impurity compound P06) was added to the trituration solvent (8.00 kg (11.7 L) of n-heptane and 2.70 kg (3 L) of ethyl acetate). The resulting mixture was triturated and stirred at 15-35 °C for 4-5 h, then centrifuged, and the filter cake was washed with water, centrifuged again, and dried. The content of the nitrosamine impurity compound P06 in the triturated compound A was detected as 0.03% (315 ppm). This illustrates that the content of the nitrosamine impurity compound P06 in compound A could be reduced by at least half by the above trituration treatment once.

The working principle of the trituration is that: the trace impurities contained in the target compound could be dissolved by the trituration solvent and left in the liquid, while the insoluble target compound is still in solid form. Then, the content of impurities in the target compound can be reduced through solid-liquid separation.

### Example 16

The liquid chromatography-tandem mass spectrometry which adopts the conditions shown in the Table 18 was used in the qualitative and/or quantitative detection of the nitrosamine impurity compound P07 in the API of varenicline tartrate in the present disclosure, and the methodological verification was carried out and passed.

**Table 18: The chromatographic and mass spectrometer conditions for measurement of the nitrosamine impurity compound P07.**

| **Parameter** | | **Specific configuration** | | |
|---|---|---|---|---|
| Chromatographic conditions | Chromatographic column | Waters Xbridge C18 (4.6mm×150mm, 3.5µm) | | |
| | Column temperature | 30°C | | |
| | Flow rate | 0.5ml/min | | |
| | Injection volume | 5µl | | |
| | Mobile phase A | 0.01mol/L ammonium acetate solution (pH=2.5, adjusted with formic acid) | | |
| | Mobile phase B | Acetonitrile | | |

| | Gradient elution | Time (min) | Mobile phase A(%) | Mobile phase B(%) |
|---|---|---|---|---|
| | | 0 | 95 | 5 |
| | | 20 | 95 | 5 |
| | | 22 | 50 | 50 |
| | | 30 | 50 | 50 |
| | | 30.1 | 95 | 5 |
| | | 40 | 95 | 5 |
| Mass spectrometer conditions | Instrument | Agilent 6410 | | |
| | Ion Source type | ESI | | |
| | Ion Source model | Positive | | |
| | Scan type | Multi Reaction Monitoring (MRM) | | |
| | Capillary voltage | 6000V | | |
| | Ion Source temp | 350°C | | |
| | Spray gas flow rate | 13L/min | | |

### 1. The preparation of solution:

Blank (diluent): Water-acetonitrile (95:5).

Control stock solution: 25mg nitrosamine compound P07 (purity≥95%) was weighed into a 50ml volumetric flask, dissolved and diluted to scale with acetonitrile and mixed evenly (volumetric solution 1). Then, 1.0ml volumetric solution I was added into a 100ml volumetric flask, diluted with the diluent to volume, and mixed evenly to obtain the control stock solution.

Control solution: 0.45ml of the control stock solution was added into a 100ml volumetric flask, diluted to volume with the diluent and mixed evenly to obtain the control solution.

Sample solution: About 100mg of the varenicline tartrate API was added into a 10ml volumetric flask, dissolved and diluted to volume with the diluent, then mixed evenly to obtain the sample solution.

### 2. System suitability:

5 needles of the control solution were injected, the RSD of the peak area of the nitrosamine impurity compound P07 was ≤15%, and the RSD of the retention time was ≤ 2.0%, which illustrated that the adjacent peaks could be separated well, and the system meant a good suitability.

### 3. Linearity

The control solutions with concentrations of 0.002688µg/ml, 0.020157µg/ml, 0.033594 µg/ml, 0.053751µg/ml, 0.067189µg/ml, and 0.100783 µg/ml (calculated as nitrosamine compound P07) were prepared respectively.

The control solutions with different concentrations were injected respectively for analysis. The linear regression was carried out, wherein the concentration of the target substance (X) was taken as the horizontal coordinate (unit: µg/ml), and the peak area (Y) was taken as the ordinate. The linear equation and linear correlation coefficient r were obtained by calculating. The results showed that the linear equation is Y=3000000X+2671.7, and the linear correlation coefficient r=0.998, which illustrated that the linearity was good in the concentration range of 0.002688µg/ml-0.100783µg/ml (i.e., 0.2688ppm-10.0783ppm).

### 4. Limit of detection (LOD):

LOD= 0.1344ppm, and the S/N (signal-to-noise ratio) of the target peak were 14.8 and 13.1, i.e., which met the requirement of greater than 3, showing that the method was sensitive.

### 5. Limit of quantitation (LOQ):

LOQ= 0.2688ppm, and the S/N of the target peak were 20.5 and 24.9, i.e., which met the requirement of greater than 10, showing that the method was sensitive.

### 6. Specificity:

The detection results showed that the retention time of the nitrosamine impurity compound P07 was about 14.487 min. There was no interference between the adjacent peaks, which met the requirements of resolution and meant a good specificity.

In addition, it has been verified that the accuracy, precision, repeatability and durability of the detection method also meet the requirements of method verification, and the method can be used for the qualitative and/or quantitative detection of nitrosamine impurity compound P07 in the varenicline tartrate API.

### Example 17

The liquid chromatography-tandem mass spectrometry which adopts the conditions shown in the Table 19 was used in the qualitative and/or quantitative detection of the nitrosamine impurity compound P06 in the API of varenicline tartrate in the present disclosure, and the methodological verification was carried out and passed.

**Table 19: The chromatographic and mass spectrometer conditions for measurement of the nitrosamine impurity compound P06.**

| Parameter | | Specific configuration | |
|---|---|---|---|
| chromatographic conditions | Chromatographic column | Waters Xbridge C18 (4.6mm×150mm, 3.5µm) | |
| | Column temperature | 30°C | |
| | Flow rate | 0.5ml/min | |
| | Injection volume | 30µl | |
| | Mobile phase A | Formic acid-purified water (0.1:100) | |
| | Mobile phase B | Acetonitrile | |
| | Isocratic elution | Mobile phase A(%) | Mobile phase B(%) |
| | | 63 | 37 |
| mass spectrometer conditions | Instrument | Agilent 6410 | |
| | Ion Source type | ESI | |
| | Ion Source model | Positive | |
| | Scan type | Single Ion Monitoring (SIM) | |
| | Capillary voltage | 6000V | |
| | Ion Source temp | 350°C | |
| | Spray gas flow rate | 13L/min | |

### 1. The preparation of solution

Blank (diluent): Purified water.

Control stock solution: 25mg nitrosamine compound P06 (purity≥92%) was weighed into a 50ml volumetric flask, dissolved and diluted with acetonitrile to volume and mixed evenly (volumetric solution II). Then, 1.0ml volumetric solution II was added into a 100ml volumetric flask, diluted with the diluent to volume, and mixed evenly to obtain the control stock solution.

Control solution: 4.5ml of the control stock solution was added into a 50ml volumetric flask, and diluted with the diluent to volume and mixed evenly to obtain the control solution.

Sample solution: 5.0ml diluent was measured accurately to dissolve 1000mg of the varenicline tartrate API in a 20ml headspace bottle and mixed evenly.

### 2. System suitability:

5 needles of the control solution were continuously injected, the RSD of the peak area of the nitrosamine impurity compound P06 was ≤ 15 %, and the RSD of the retention time was ≤ 2.0%, which illustrated that the adjacent peaks could be separated well, indicating that the system had a good suitability.

### 3. Linearity

The control solutions with different concentrations were injected respectively for analysis. The linear regression was carried out, wherein the concentration of the target substance (X) was taken as the horizontal coordinate (unit: µg/ml), and the peak area (Y) was taken as the ordinate. The linear equation and linear correlation coefficient r were obtained by calculating. The results showed that the linear correlation coefficient r>0.99, which illustrated that the linearity was great.

### 4. Limit of detection (LOD):

LOD= 0.0145ppm, and the S/N (signal-to-noise ratio) of the target peak was 5.8, which met the requirement of greater than 3, showing that the method was sensitive.

### 5. Limit of quantitation (LOQ):

LOQ= 0.0289ppm, and the S/N of the target peak was 12, which met the requirement of greater than 10, showing that the method was sensitive.

### 6. Specificity:

The detection results showed that the retention time of the nitrosamine impurity compound P06 was about 14.648 min. There was no interference between the adjacent peaks, which met the requirements of resolution and meant a good specificity.

Moreover, it has been verified that the accuracy, precision, repeatability and durability and the like of the detection method also meet the requirements of the method verification, and the method can be used for the qualitative and/or quantitative detection of nitrosamine impurity compound P06 in the varenicline tartrate API.

### Example 18

In order to better ensure the limit requirements for nitrosamine impurity compounds (including: P06, P07, P08; wherein P08 is transformed from P06 via the intermediate product P07; therefore, P08 is a main form of nitrosamine impurities in varenicline tartrate API or formulation, P06 is a main form of nitrosamine impurities in the raw materials such as compound A, and P07 is an intermediate product during the preparation process) in varenicline tartrate API or formulation, it is necessary to perform qualitative and/or quantitative detection (for example by HPLC) of nitrosamine impurity compound P06 in the initial compound A. The chromatographic conditions thereof are shown in Table 20.

**Table 20: The chromatographic conditions for measurement of the nitrosamine impurity compound P06 in compound A.**

| Parameter | | Specific configuration | |
|---|---|---|---|
| Chromatographic conditions | Chromatographic column | Kromasil 100-5 C18 (4.6mm×250mm, 5µm) | |
| | Column temperature | 30°C | |
| | Flow rate | 1ml/min | |
| | Detection wavelength | 270nm | |
| | Injection volume | 10µl | |
| | Mobile phase A | 0.05% phosphoric acid aqueous solution | |
| | Mobile phase B | methanol | |
| | Isocratic elution | Mobile phase A (%) | Mobile phase B (%) |
| | | 40 | 60 |

### 1. The preparation of standard curve:

Blank (diluent): acetonitrile-water (60:40).

Control solution: 25mg nitrosamine compound P06 (purity ≥92%) was weighed into a 25ml volumetric flask, diluted with diluent to volume and mixed evenly to obtain the control solution.

The control solutions with concentrations of 0.0001394mg/ml, 0.0004645mg/ml, 0.0007432mg/ml, 0.000929 mg/ml, 0.001115mg/ml, 0.001394mg/ml and 0.001858mg/ml were prepared respectively.

The control solutions with different concentrations were injected respectively for analysis. The linear regression was carried out, wherein the concentration of the target substance (X) was taken as the horizontal coordinate (unit: mg/ml), and the peak area (Y) was taken as the ordinate. The linear equation and linear correlation coefficient r were obtained by calculating. The results showed that the linear equation is Y=228.94X+0.0025, and the linear correlation coefficient r is 0.999, which illustrates that the linearity was good in the above concentration ranges.

Limit of detection: LOD = 0.004% (40ppm), and the S/N (signal-to-noise ratio) of the target peak was 6.4, which met the requirement of greater than 3, showing that the method was sensitive.

Limit of quantitation: LOQ = 0.014% (140ppm), and the S/N of the target peak was 18.9, which met the requirement of greater than 10, showing that the method was sensitive.

Specificity: The detection results of the mixed solution of the sample and the control solutions showed that the retention time of the nitrosamine impurity compound P06 was about 4.592 min (resolution R = 13.18), the retention time of the compound A was about 7.587 min (resolution R = 6.82), the retention time of the impurity M01 was about 9.373 min (resolution R = 7.24), and the retention time of the impurity M02 was about 12.45 min (resolution R = 5.25). There was no interference between the peaks, which met the requirements of resolution and meant a good specificity.

In addition, it has been verified that the accuracy, precision, repeatability and durability and the like of the detection method also meet the requirements of method verification, and the method can be used for the qualitative and/or quantitative detection of nitrosamine impurity compound P06 in compound A.

### 2. The detection of the sample:

Sample solution: About 25mg compound A was weighed into a 25ml volumetric flask, diluted to volume with the diluent and mixed evenly.

The sample solution was injected for detection, and the peak area data and the chromatographic spectrum were obtained (see, Fig 1). The peak area data were substituted into the above linear equation to calculate and obtain the concentration data of the nitrosamine impurity compound P06 in the sample solution.

Then, the content of the nitrosamine impurity compound P06 in the sample (compound A) was calculated according to the following formula: The content of P06: C (expressed in % or ppm) = the concentration of P06 in the sample solution (mg/ml)/ [m (mg)/V(ml)];
m: sample mass, mg;
V: sample dilution volume, ml.

Of course, the present disclosure may have other various embodiments. Those skilled in the art may make various corresponding changes and/or modifications according to the present disclosure without departing from the spirit and essence thereof, and these corresponding changes and/or modifications shall fall within the protection scope of the appended claims.

## Claims

1. A pharmaceutical composition, comprising a secondary amine compound and a pharmaceutically acceptable acid; wherein the secondary amine compound is varenicline or a pharmaceutically acceptable salt thereof.

2. The pharmaceutical composition according to claim 1, wherein the secondary amine compound is the pharmaceutically acceptable salt of varenicline.

3. The pharmaceutical composition according to claim 2, wherein the secondary amine compound is varenicline hydrochloride or varenicline tartrate.

4. The pharmaceutical composition according to any one of claims 1-3, wherein the secondary amine compound and the pharmaceutically acceptable acid are at a molar ratio of 1: (0.01-50).

5. The pharmaceutical composition according to claim 4, wherein the secondary amine compound and the pharmaceutically acceptable acid are at a molar ratio of 1: (1-20).

6. The pharmaceutical composition according to claim 5, wherein the secondary amine compound and the pharmaceutically acceptable acid are at a molar ratio of 1: (2-10).

7. The pharmaceutical composition according to any one of claims 1-3, wherein the active ingredient of the pharmaceutical composition is varenicline, and the pharmaceutical composition further comprises pharmaceutically acceptable excipients.

8. The pharmaceutical composition according to claim 7, wherein the pharmaceutically acceptable excipients comprise one or more of the following excipients:
(1) Diluent, wherein the diluent is selected from the group consisting of one or more of microcrystalline cellulose, silicified microcrystalline cellulose, dibasic calcium phosphate, mannitol, copovidone, hydroxypropyl cellulose, hydroxypropyl methyl cellulose, methyl cellulose, ethyl cellulose, starch, maltodextrin, agar, and guargum;
(2) Disintegrant, wherein the disintegrant is selected from the group consisting of one or more of croscarmellose sodium, sodium starch glycolate, crospovidon, partially prepasted starch, partially pregelatinized starch, pregelatinized starch, pregelatinized hydroxypropyl starch, sodium carboxymethyl cellulose, and calcium carboxymethyl cellulose;
(3) Glidant, wherein the glidant is selected from the group consisting of one or more of colloidal silicon dioxide, fumed silicon dioxide, colloidal silica, corn starch, talc, calcium silicate, magnesium silicate, calcium phosphate tribasic, and silicone hydrogel;
(4) Lubricant, wherein the lubricant is selected from the group consisting of one or more of stearic acid, stearate, talc, mineral oil, malt, glyceryl monostearate, glyceryl benzoate, glyceryl stearate palmitin, hydrogenated vegetable oil, polyethylene glycol, sodium benzoate, sodium acetate, sodium chloride, leucine, lauryl magnesium sulfate, and lauryl sodium sulfate;
(5) Coating agent, wherein the coating agent is Opadry.

9. The pharmaceutical composition according to claim 8, wherein the pharmaceutically acceptable excipients simultaneously comprise the excipients of (1)-(4) or (1)-(5).

10. The pharmaceutical composition according to any one of claims 1-3, wherein the pharmaceutical composition is a solid formulation or a liquid formulation, and the pharmaceutical composition is an oral formulation.

11. The pharmaceutical composition according to claim 10, wherein the solid formulation is a tablet.

12. The pharmaceutical composition according to claim 10, wherein under the following measuring conditions, the pharmaceutical composition has a pH value of ≤ 4; when the pharmaceutical composition is a solid formulation, the measuring conditions comprise: a dispersion with a concentration of 20%-30% is obtained by mixing the pharmaceutical composition with water (pH=6 ± 0.1), then the pH value of the dispersion is measured; when the pharmaceutical composition is liquid formulation, the measuring conditions comprise: a solvent of the liquid formulation is water (pH=6 ± 0.1), and the concentration of the liquid formulation is 20%-30% (g/ml), then the pH value of the liquid formulation is measured.

13. The pharmaceutical composition according to claim 12, wherein under the following measuring conditions, the pharmaceutical composition has a pH value of 1 to 4; when the pharmaceutical composition is a solid formulation, the measuring conditions comprise: a dispersion with a concentration of 20%-30% is obtained by mixing the pharmaceutical composition with water (pH=6 ± 0.1), then the pH value of the dispersion is measured; when the pharmaceutical composition is liquid formulation, the measuring conditions comprise: a solvent of the liquid formulation is water (pH=6 ± 0.1), and the concentration of the liquid formulation is 20%-30% (g/ml), then the pH value of the liquid formulation is measured.

14. The pharmaceutical composition according to any one of claims 1-3, wherein the pharmaceutically acceptable acid is a solid acid or a liquid acid.

15. The pharmaceutical composition according to claim 14, wherein the solid acid is selected from the group consisting of one or more of tartaric acid, succinic acid, maleic acid, fumaric acid, citric acid, malic acid, ascorbic acid, benzenesulfonic acid, oxalic acid, triphenylacetic acid, 1-hydroxyl-2-naphthalenemethyl acid, and 3-hydroxyl-2-naphthalenemethyl acid; wherein the liquid acid is selected from the group consisting of one or more of hydrochloric acid, sulfuric acid, phosphoric acid, hydrofluoric acid, hydrobromic acid, acetic acid, trifluoroacetic acid, propanoic acid, methanesulfonic acid, and trifluoromethanesulfonic acid.

16. The pharmaceutical composition according to claim 14, wherein the pharmaceutically acceptable acid is a solid acid.

17. The pharmaceutical composition according to claim 16, wherein the pharmaceutically acceptable acid is tartaric acid.

18. The pharmaceutical composition according to claim 8, wherein the pharmaceutical composition comprises the following components in parts of weight: 1.71 parts of the secondary amine compounds; 100-250 parts of the diluent; 2-10 parts of the disintegrant; 0-5 parts of the glidant; 0-5 parts of the lubricant; and 0.01-15 parts of the pharmaceutically acceptable acid.

19. The pharmaceutical composition according to claim 18, wherein the pharmaceutical composition comprises the following components in parts by weight: 1.71 parts of the secondary amine compounds; 184.47 parts of the diluent; 4 parts of the disintegrant; 0.6 parts of the glidant; 5 parts of the lubricant; and 1.71-3.42 parts of pharmaceutically acceptable acid.

20. The pharmaceutical composition according to claim 19, wherein the pharmaceutical composition is any one of the following formulations:
Formulations 1:
The pharmaceutical composition is composed of the following components in parts by weight: 1.71 parts of the varenicline tartrate; 64 parts of the anhydrous calcium bicarbonate; 120.47 parts of the microcrystalline cellulose; 4 parts of the partially pregelatinized maize starch; 0.6 part of the colloidal silicon dioxide; 5 parts of the stearic acid; and 3.42 parts of the L (+) tartaric acid;
Formulations 2:
The pharmaceutical composition is composed of the following components in parts by weight: 1.71 parts of the varenicline tartrate; 64 parts of the anhydrous calcium bicarbonate; 120.47 parts of the microcrystalline cellulose; 4 parts of the partially pregelatinized maize starch; 0.6 part of the colloidal silicon dioxide; 5 parts of the stearic acid; and 1.71 parts of the L (+) tartaric acid;
Formulations 3:
The pharmaceutical composition is composed of the following components in parts by weight: 1.71 parts of the varenicline tartrate; 64 parts of the anhydrous calcium bicarbonate; 120.47 parts of the microcrystalline cellulose; 4 parts of the partially pregelatinized maize starch; 0.6 part of the colloidal silicon dioxide; 5 parts of the stearic acid; 3.42 parts of the L (+) tartaric acid; and 6 parts of the Opadry-white or Opadry-blue; or
Formulations 4:
The pharmaceutical composition is composed of the following components in parts by weight: 1.71 parts of the varenicline tartrate; 64 parts of the anhydrous calcium bicarbonate; 120.47 parts of the microcrystalline cellulose; 4 parts of the partially pregelatinized maize starch; 0.6 part of the colloidal silicon dioxide; 5 parts of the stearic acid; 1.71 parts of the L (+) tartaric acid; and 6 parts of the Opadry-white or Opadry-blue.

21. The pharmaceutical composition according to any one of claims 1-3, wherein the content of the nitrosamine impurities in the pharmaceutical composition is not more than 7.5 ppm; wherein the nitrosamine impurity is:

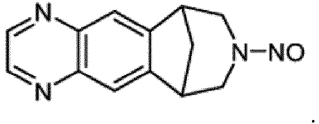

22. The pharmaceutical composition according to claim 21, wherein the content of the nitrosamine impurities in the pharmaceutical composition is not more than 5.0 ppm.

23. The pharmaceutical composition according to claim 21, wherein the content of the nitrosamine impurities in the pharmaceutical composition is not more than 4.0 ppm.

24. The pharmaceutical composition according to claim 21, wherein the content of the nitrosamine impurities in the pharmaceutical composition is not more than 3.0 ppm.

25. A method for preparing a pharmaceutical composition according to any one of claims 1-24, comprising the steps of mixing all of the components and then granulating and tableting, and then optionally comprising a coating step to obtain the final product.

26. The method for preparing a pharmaceutical composition according to claim 25, wherein the method comprise the steps of screening the colloidal silicon dioxide, anhydrous dibasic calcium phosphate, varenicline tartrate and the pharmaceutically acceptable acid with a 40-mesh sieve, mixing evenly, then adding microcrystalline cellulose, partially pregelatinized maize starch, and intra-granular stearic acid, mixing evenly and granulating, then adding extra-granular stearic acid, mixing evenly and tableting, and then optionally comprises a coating step to obtain final product.

27. The process for preparing a pharmaceutical composition according to claim 26, wherein the granulating is a dry granulation or a wet granulation, and wherein the tableting is a direct compression process.

28. A use of a pharmaceutical composition according to any one of claims 1-24 in the preparation of a medicament; the pharmaceutical composition is used to prevent or treat the following diseases: inflammatory bowel disease, ulcerative colitis, pyoderma gangrenosum, Crohn's disease, irritable bowel syndrome, spastic dystonia, chronic pain, acute pain, celiac sprue, pouchitis, vasoconstriction, anxiety, panic disorder, depression, bipolar disorder, autism, sleep disorders, jet lag, amyotrophic lateral sclerosis, cognitive dysfunction, cognitive impairment caused by alcohol, barbiturates, vitamin deficiencies, recreational drugs, lead, arsenic, or mercury; Alzheimers disease, senile dementia, vascular dementia, Parkinson's disease, multiple sclerosis, AIDS, encephalitis, trauma, hepatic and renal encephalopathy, hypothyroidism, Pick's disease, Korsakoff s syndrome, cognitive impairment caused by frontal dementia or subcortical dementia, hypertension, bulimia, anorexia, obesity, cardiac arrhythmias, gastric acid hypersecretion, ulcers, pheochromocytoma, progressive supramuscular palsy, chemical dependencies on and addictions to nicotine, tobacco products, alcohol, benzodiazepines, barbiturates, opioids or cocaine, headache, migraine, stroke, traumatic brain injury, obsessive-compulsive disorder, psychosis, Huntington's chorea, tardive dyskinesia, hyperkinesia, dyslexia, schizophrenia, multi-infarct dementia, age-related cognitive decline, epilepsy, attention deficit hyperactivity disorder, or Tourette's Syndrome.

29. The use of a pharmaceutical composition in the preparation of a medicament according to claim 28, wherein the medicament meets any one of the following conditions of (a) to (c):
(a). The medicament is used to treat dependencies on, addictions to or deaddictions to nicotine;
(b). The medicament is used to treat dependencies on, addictions to or deaddictions to tobacco products; and
(c). The medicament is a smoking cessation medicament.

30. A use of pharmaceutically acceptable acid to inhibit or retard the nitrosamineization of secondary amine compound, wherein the secondary amine compound is varenicline or the pharmaceutically acceptable salt thereof.

31. The use of a pharmaceutically acceptable acid to inhibit or retard the nitrosamineization of secondary amine compound according to claim 30, wherein the use of a pharmaceutically acceptable acid to inhibit or retard the nitrosamineization of secondary amine compound meets one or more of the following conditions of Ia to Id:
Ia. The secondary amine compound and the pharmaceutically acceptable acid are at a molar ratio of 1: (0.01-50);
Ib. The secondary amine compound is the pharmaceutically acceptable salt of varenicline;
Ic. The pharmaceutically acceptable acid is a solid acid or a liquid acid, and the solid acid is selected from the group consisting of one or more of tartaric acid, succinic acid, maleic acid, fumaric acid, citric acid, malic acid, ascorbic acid, benzenesulfonic acid, oxalic acid, triphenylacetic acid, 1-hydroxyl-2-naphthalenemethyl acid, and 3-hydroxyl-2-naphthalenemethyl acid; wherein the liquid acid is selected from the group consisting of one or more of hydrochloric acid, sulfuric acid, phosphoric acid, hydrofluoric acid, hydrobromic acid, acetic acid, trifluoroacetic acid, propanoic acid, methanesulfonic acid, and trifluoromethanesulfonic acid; and
Id. The addition amount of the pharmaceutically acceptable acid is such that the pH value of mixture X under the following measuring conditions is ≤ 4; wherein the mixture X comprises the pharmaceutically acceptable acid and the secondary amine compound; when the mixture X is a solid, the measuring conditions comprise: a dispersion with a concentration of 20%-30% is obtained by mixing the mixture X with water (pH=6 ± 0.1), then the pH value of the dispersion is measured; when the mixture X is a liquid, the measuring conditions comprise: the solvent of the liquid is water (pH = 6 ± 0.1), and the concentration of the liquid is 20%-30%, then the pH value of the liquid is measured.

32. The use of a pharmaceutically acceptable acid to inhibit or retard the nitrosamineization of secondary amine compound according to claim 31, wherein the addition amount of the pharmaceutically acceptable acid is such that the pH value of mixture X under the following measuring conditions is 1-4; wherein the mixture X comprises the pharmaceutically acceptable acid and the secondary amine compound; when the mixture X is a solid, the measuring conditions comprise: a dispersion with a concentration of 20%-30% is obtained by mixing the mixture X with water (pH=6 ± 0.1), then the pH value of the dispersion is measured; when the mixture X is a liquid, the measuring conditions comprise: the solvent of the liquid is water (pH = 6 ± 0.1), and the concentration of the liquid is 20%-30%, then the pH value of the liquid is measured.

33. The use of a pharmaceutically acceptable acid to inhibit or retard the nitrosamineization of secondary amine compound according to claim 31, wherein the use of a pharmaceutically acceptable acid to inhibit or retard the nitrosamineization of secondary amine compound simultaneously meets the conditions of Ia-Id.

34. The use of a pharmaceutically acceptable acid to inhibit or retard the nitrosamineization of secondary amine compound according to claim 31, wherein the use of a pharmaceutically acceptable acid to inhibit or retard the nitrosamineization of secondary amine compound meets one or more of the conditions of IIa-IIc:
IIa. The secondary amine compound and the pharmaceutically acceptable acid are at a molar ratio of 1: (1-20);
IIb. The secondary amine compound is varenicline hydrochloride or varenicline tartrate; and
IIc. The pharmaceutically acceptable acid is a solid acid.

35. The use of a pharmaceutically acceptable acid to inhibit or retard the nitrosamineization of secondary amine compound according to claim 34, wherein the secondary amine compound and the pharmaceutically acceptable acid are at a molar ratio of 1: (2-10); and the pharmaceutically acceptable acid is tartaric acid.

36. The use of a pharmaceutically acceptable acid to inhibit or retard the nitrosamineization of secondary amine compound according to claim 34, wherein the use of a pharmaceutically acceptable acid to inhibit or retard the nitrosamineization of secondary amine compound simultaneously meets the conditions of IIa-IIc.

37. The use of a pharmaceutically acceptable acid to inhibit or retard the nitrosamineization of secondary amine compound according to claim 34, wherein the inhibition or retardation of the nitrosamineization of secondary amine compound is the inhibition or retardation of the nitrosaminization of the secondary amine compound to generate

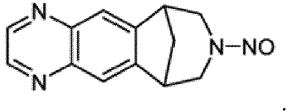

38. A method for inhibiting or retarding the nitrosamineization of secondary amine compound, comprising mixing the secondary amine compound or the composition thereof with the pharmaceutically acceptable acid evenly; wherein the secondary amine compound is varenicline or the pharmaceutically acceptable salt thereof.

39. The method for inhibiting or retarding the nitrosamineization of secondary amine compound according to claim 38, wherein the method for inhibiting or retarding the nitrosamineization of secondary amine compound comprises the steps of adding the pharmaceutically acceptable acid into the secondary amine compound or the composition thereof, and mixing evenly.

40. The method for inhibiting or retarding the nitrosamineization of secondary amine compound according to claim 38, wherein the composition of the secondary amine compound comprises all of the components of the pharmaceutical composition according to any one of claims 1-24, except the pharmaceutically acceptable acid.

41. The method for inhibiting or retarding the nitrosamineization of secondary amine compound according to claim 38, wherein the method for inhibiting or retarding the nitrosamineization of secondary amine compound meets one or more of the conditions Ia-Id:
Ia. The secondary amine compound and the pharmaceutically acceptable acid are at a molar ratio of 1: (0.01-50);
Ib. The secondary amine compound is the pharmaceutically acceptable salt of varenicline;
Ic. The pharmaceutically acceptable acid is a solid acid or a liquid acid, and the solid acid is selected from the group consisting of one or more of tartaric acid, succinic acid, maleic acid, fumaric acid, citric acid, malic acid, ascorbic acid, benzenesulfonic acid, oxalic acid, triphenylacetic acid, 1-hydroxyl-2-naphthalenemethyl acid, and 3-hydroxyl-2-naphthalenemethyl acid; wherein the liquid acid is selected from the group consisting of one or more of hydrochloric acid, sulfuric acid, phosphoric acid, hydrofluoric acid, hydrobromic acid, acetic acid, trifluoroacetic acid, propanoic acid, methanesulfonic acid, and trifluoromethanesulfonic acid; and
Id. The addition amount of the pharmaceutically acceptable acid is such that the pH value of mixture X under the following measuring conditions is ≤ 4; wherein the mixture X comprises the pharmaceutically acceptable acid and the secondary amine compound; when the mixture X is a solid, the measuring conditions comprise: a dispersion with a concentration of 20%-30% is obtained by mixing the mixture X with water (pH=6 ± 0.1), then the pH value of the dispersion is measured; when the mixture X is a liquid, the measuring conditions comprise: the solvent of the liquid is water (pH = 6 ± 0.1), and the concentration of the liquid is 20%-30%, then the pH value of the liquid is measured.

42. The method for inhibiting or retarding the nitrosamineization of secondary amine compound according to claim 41, wherein the addition amount of the pharmaceutically acceptable acid is such that the pH value of mixture X under the following measuring conditions is 1-4; wherein the mixture X comprises the pharmaceutically acceptable acid and the secondary amine compound; when the mixture X is a solid, the measuring conditions comprise: a dispersion with a concentration of 20%-30% is obtained by mixing the mixture X with water (pH=6 ± 0.1), then the pH value of the dispersion is measured; when the mixture X is a liquid, the measuring conditions comprise: the solvent of the liquid is water (pH = 6 ± 0.1), and the concentration of the liquid is 20%-30%, then the pH value of the liquid is measured.

43. The method for inhibiting or retarding the nitrosamineization of secondary amine compound according to claim 41, wherein the method for inhibiting or retarding the nitrosamineization of secondary amine compound simultaneously meets the conditions of Ia-Id.

44. The method for inhibiting or retarding the nitrosamineization of secondary amine compound according to claim 41, wherein the method for inhibiting or retarding the nitrosamineization of secondary amine compound meets one or more of the conditions IIa-IIc:
IIa. The secondary amine compound and the pharmaceutically acceptable acid are at a molar ratio of 1: (1-20);
IIb. The secondary amine compound is varenicline hydrochloride or varenicline tartrate; and
IIc. The pharmaceutically acceptable acid is a solid acid.

45. The method for inhibiting or retarding the nitrosamineization of secondary amine compound according to claim 44, wherein the secondary amine compound and the pharmaceutically acceptable acid are at a molar ratio of 1: (2-10); and the pharmaceutically acceptable acid is tartaric acid.

46. The method for inhibiting or retarding the nitrosamineization of secondary amine compound according to claim 44, wherein the method for inhibiting or retarding the nitrosamineization of secondary amine compound simultaneously meets the conditions IIa-IIc.

47. The method for inhibiting or retarding the nitrosamineization of secondary amine compound according to claim 44, wherein the inhibition or retardation of the nitrosamineization of secondary amine compound is the inhibition or retardation of the nitrosaminization of the secondary amine compound to generate

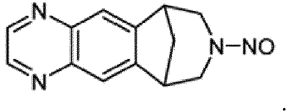

48. A compound of formula P08, the pharmaceutically acceptable salt, crystal form, solvate thereof or solvate of the pharmaceutically acceptable salt thereof:

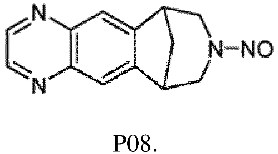

49. The use of a compound of formula P08, the pharmaceutically acceptable salt, crystal form, solvate thereof or solvate of the pharmaceutically acceptable salt thereof as insecticide:

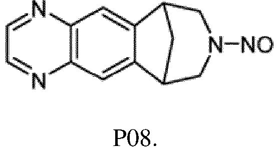

50. The use of the compound of formula P08, the pharmaceutically acceptable salt, crystal form, solvate thereof or solvate of the pharmaceutically acceptable salt thereof as insecticide according to claim 49, wherein the insecticide is used for controlling Tetranychus cinnabarinus.

51. A use of a compound of formula P08 as a standard substance, a control substance, or a detection item in impurity studies, quality control or measuring methods for active pharmaceutical ingredients or formulation of secondary amine compound; wherein the secondary amine compound is varenicline or a pharmaceutically acceptable salt thereof, and wherein the measuring method is liquid chromatography-tandem mass spectrometry:
